(19) Europäisches Patentamt
European Patent Office
Office européen des brevets

(11) **EP 3 492 102 A1**

(12) **EUROPEAN PATENT APPLICATION**

(43) Date of publication:
    **05.06.2019 Bulletin 2019/23**

(51) Int Cl.:
    ***A61K 41/00*** *(2006.01)*

(21) Application number: **17020555.3**

(22) Date of filing: **30.11.2017**

(84) Designated Contracting States:
    **AL AT BE BG CH CY CZ DE DK EE ES FI FR GB
    GR HR HU IE IS IT LI LT LU LV MC MK MT NL NO
    PL PT RO RS SE SI SK SM TR**
    Designated Extension States:
    **BA ME**
    Designated Validation States:
    **MA MD**

(71) Applicant: **Nanobacterie
    75116 Paris (FR)**

(72) Inventor: **ALPHANDERY, Edouard
    75116 Paris (FR)**

(54) **NANOPARTICLES EXPOSED SEQUENTIALLY TO LOW INTENSITY ACOUSTIC WAVES FOR MEDICAL TREATMENT**

(57)    This invention relates to nanoparticles for use in an acoustic wave medical treatment of a body part of an individual, wherein the nanoparticles possess a specific absorption rate larger than $10^{-9}$ Watt per gram of nanoparticle and the nanoparticles are administered to the body part.

**EP 3 492 102 A1**

**Description**

**Field of the invention**

This invention is related to nanoparticles being sequentially exposed to low intensity acoustic waves for medical treatment.

**Technical background**

Diseases such as cancers, for example prostate cancer, can currently be treated with ultrasounds, usually using high intensity focused ultrasounds (HIFU). However, this treatment method presents several drawbacks. To be efficient, HIFU usually requires to heat cancer tissues at relatively high temperatures, which can be damaging for healthy tissues. Using HIFU often necessitates heating the tumor in several spots to eradicate it. To overcome these drawbacks, low intensity ultrasound (LIU) could be used instead of HIFU. In this invention, we propose a method to make LIU efficient for medical treatment. It is based on the use of nanoparticles located in a pathological site, which are sequentially exposed to acoustic waves of low intensity.

**Description of the Figures**

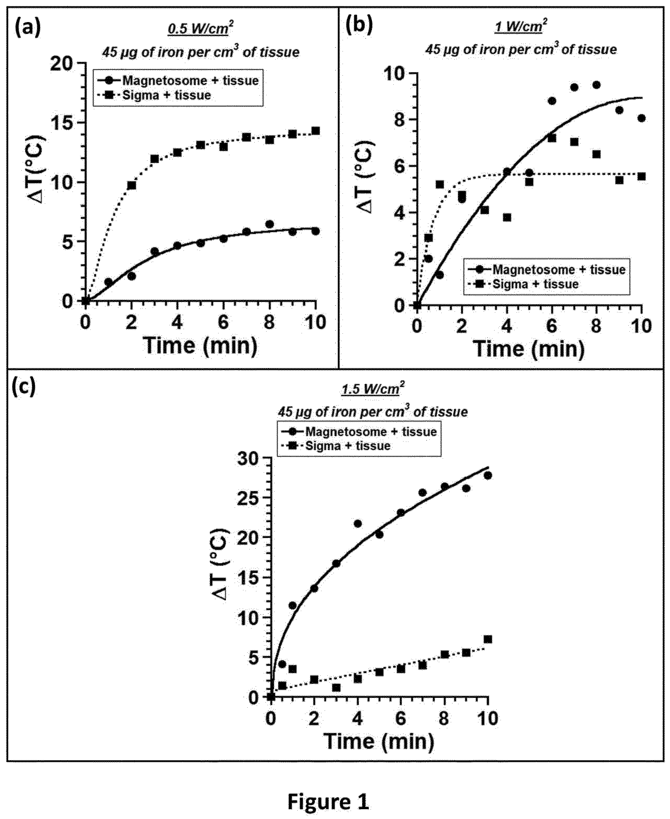

Figure 1: (a), For 210 $\mu$g in iron of nanoparticles (magnetosomes or Sigma) inserted in 4.6 cm$^3$ of tissue exposed to ultrasounds of frequency 3 MHz and power 0.5 W/cm$^2$, $\Delta$T, designing the temperature difference between the temperature measured for the tissue with the nanoparticles and the temperature measured for the tissue without the nanoparticles, as a function of time. (b), same as in (a) for an ultrasound power of 1 W/cm$^2$. (c), same as in (a) for an ultrasound power of 1.5 W/cm$^2$.

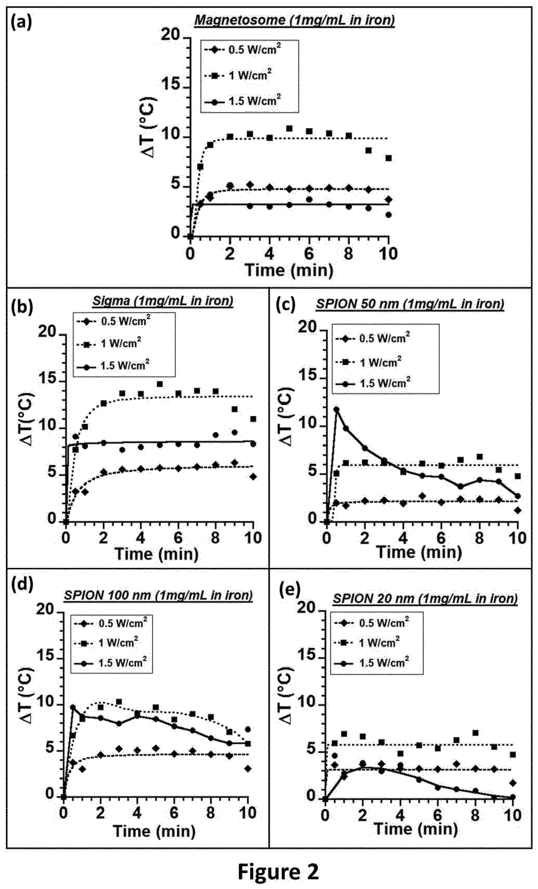

Figure 2: (a), For 100 $\mu$g in iron of magnetosomes dispersed in 100 $\mu$l of water exposed to ultrasounds of frequency 3 MHz and power 0.5 W/cm$^2$, 1 W/cm$^2$, or 1.5 W/cm$^2$, $\Delta$T, designing the temperature difference between the temperature measured for magnetosomes dispersed in water and the temperature measured for water without magnetosomes, as a function of time. (b), For 100 $\mu$g in iron of Sigma dispersed in 100 $\mu$l of water exposed to ultrasounds of frequency 3 MHz and power 0.5 W/cm$^2$, 1 W/cm$^2$, or 1.5 W/cm$^2$, $\Delta$T, designing the temperature difference between the temperature measured for Sigma dispersed in 100 $\mu$l of water and the temperature measured for 100 $\mu$l of water without Sigma, as a function of time. (c), For 100 $\mu$g in iron of SPION50 dispersed in 100 $\mu$l of water exposed to ultrasounds of frequency 3 MHz and power 0.5 W/cm$^2$, 1 W/cm$^2$, or 1.5 W/cm$^2$, $\Delta$T, designing the temperature difference between the temperature measured for SPION50 dispersed in 100 $\mu$l of water and the temperature measured for 100 $\mu$l of water without SPION50, as a function of time. (d), For 100 $\mu$g in iron of SPION100 dispersed in 100 $\mu$l of water exposed to ultrasounds of frequency 3 MHz and power 0.5 W/cm$^2$, 1 W/cm$^2$, or 1.5 W/cm$^2$, $\Delta$T, designing the temperature difference between the temperature measured for SPION100 dispersed in 100 $\mu$l of water and the temperature measured for 100 $\mu$l of water without SPION100, as a function of time. (e), For 100 $\mu$g in iron of SPION20 dispersed in 100 $\mu$l of water exposed to ultrasounds of frequency 3 MHz and power 0.5 W/cm$^2$, 1 W/cm$^2$, or 1.5 W/cm$^2$, $\Delta$T, designing the temperature difference between the temperature measured for SPION20 dispersed in 100 $\mu$l of water and the temperature measured for 100 $\mu$l of water without SPION20, as a function of time.

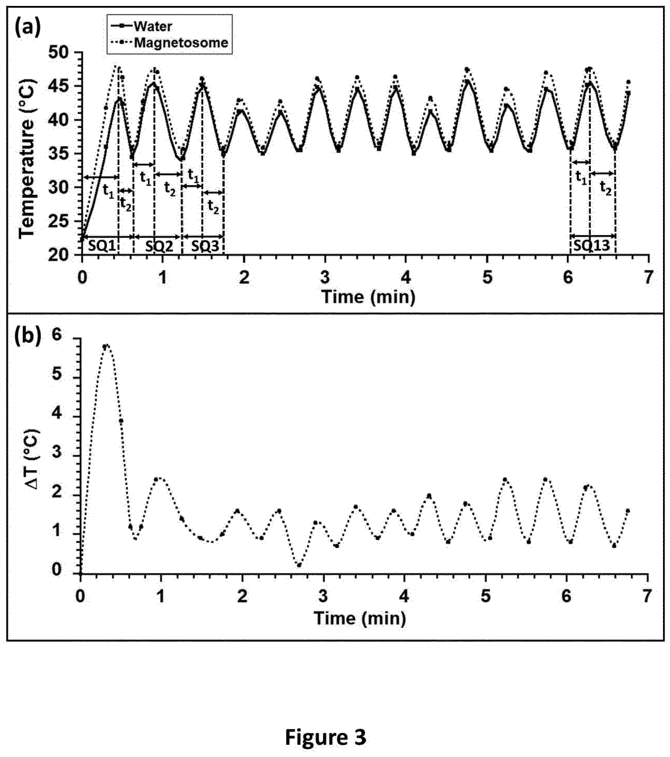

Figure 3: (a), For 800 $\mu$g in iron of magnetosomes dispersed in 100 $\mu$l of water (magnetosomes) or 100 $\mu$l of water without magnetosomes (water) exposed to ultrasounds of frequency 3 MHz and power 1.5 W/cm$^2$ during heating steps of duration $t_1$ and no exposed to ultrasounds during cooling steps of duration $t_2$, where the different values of heating and cooling times ($t_1$ and $t_2$) during sequences S1 to S13 are indicated in table 3. (b), Difference between the temperature of magnetosomes dispersed in water (Magnetosome in (a)) and the temperature of water without the magnetosomes (Water in (b)) as a function of time during the different sequences.

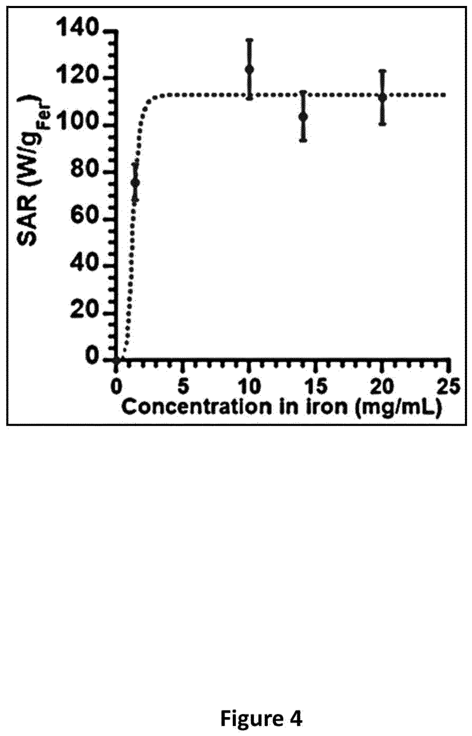

Figure 4: For 100 $\mu$l of a suspension of BNF-Starch nanoparticles (ref Micromod: 10-00102) mixed in water, exposed to an alternating magnetic field of average strength 30 mT and frequency 196 kHz, variation of the SAR, expressed in watt per gram of iron comprised in nanoparticles, as a function of the iron concentration comprised in BNF-Starch. BNF-Starch are ferrimagnetic iron oxide nanoparticles of average sizes 18 nm.

**Description of the invention**

In this invention, an acoustic wave can be defined as: i), a mechanical wave, which preferentially induces a mechanical perturbation of the medium or body part through which it travels such as compression and/or expansion of the medium, or ii), a wave that induces or is associated with the movement or vibration of a substance, atom, ion, nanoparticle with a non-zero mass or non-zero weight. The acoustic wave is usually not an electromagnetic wave. In some cases, it can however produce or generate an electromagnetic wave, for example if moving/vibrating substances associated to the acoustic wave possess a non-zero charge. The word "*acoustic wave*" can designate acoustic wave energy, acoustic wave power, acoustic wave intensity, or acoustic wave frequency. In some cases, acoustic wave intensity can have a similar meaning as acoustic wave power or acoustic wave energy. In other cases, acoustic wave power can have a similar meaning as acoustic wave energy. The acoustic wave can be absorbed by, reflected by, or transmitted through nanoparticles or body part. The acoustic wave can have a frequency, energy, power, or intensity, which can be designated as the acoustic wave frequency, energy, power, or intensity, respectively. The acoustic wave can designate an assembly of more than 1, 10, $10^2$, $10^3$, $10^5$, $10^{10}$, or $10^{20}$ acoustic wave(s). The acoustic wave energy can represent the acoustic wave power multiplied by the time of application of the acoustic wave. It can be expressed in a power unit such as Watt multiplied by a time unit such as second. The energy density of the acoustic wave can represent the energy of the acoustic wave per unit length such as cm, surface such as $cm^2$, or volume such as $cm^3$. The acoustic wave power can be proportional to the acoustic wave energy per unit time. It can be expressed in a power unit such as Watt. The acoustic wave power density can represent the acoustic wave power per unit length such as cm, per unit surface such as $cm^2$, or per unit volume such as $cm^3$. The acoustic wave intensity can be proportional to the acoustic wave power per unit surface such as $cm^2$. It can be expressed in a power unit such as Watt divided by a surface unit such as $cm^2$. In some cases, the unit length, unit surface, and unit volume can represent the length, surface, and volume of the nanoparticle(s), respectively.

In this invention, an acoustic wave can be defined as an infrasound, a sound, an ultrasound, or a hypersound. An infrasound can preferentially be defined as an acoustic wave of low frequency, preferentially of frequency lower than 2, 20, or 200 Hz. A sound can preferentially be defined as a sound of frequency larger than 2, 20, or 200 Hz and/or lower than 2, 20, or 200 kHz. An ultrasound can preferentially be defined as a sound of frequency larger than 2, 20, or 200 kHz and/or lower than 0.1, 1, or 10 GHz. A hyper-sound can preferentially be defined as a sound of frequency larger than $10^{-3}$, $10^{-2}$, $10^{-1}$, 1, or 10 GHz.

The invention relates to nanoparticles for use in an acoustic wave medical treatment of a body part of an individual, wherein the nanoparticles possess a specific absorption rate larger than $10^{-9}$ Watt per gram of nanoparticle and the nanoparticles are administered to the body part. The invention also relates to a method for treating a body part of an individual, comprising administering an effective amount of nanoparticles to the body part and applying an effective acoustic wave to the body part, wherein the nanoparticles possess a specific absorption rate larger than $10^{-9}$ Watt per gram of nanoparticles.

In one embodiment of the invention, the nanoparticles possess a specific absorption rate (SAR), which is larger or lower than $10^{-9}$, $10^{-6}$, $10^{-3}$, 1, $10^3$, $10^6$, or $10^9$ Watt per gram of nanoparticles ($W/g_{nano}$) or $10^9$ Watt per $cm^3$ of nanoparticles ($W/cm^3_{nano}$) or $10^9$ Watt per gram of body part ($W/g_{bp}$) or $10^9$ Watt per $cm^3$ of body part ($W/cm^3_{bp}$).

The lowest values of the SAR can be justified by the fact that SAR values decrease with decreasing nanoparticle concentration or nanoparticle number, preferentially by a factor of more or less than 1.1, 2, 5, 10, $10^2$, $10^3$, $10^5$, or $10^{10}$, when the nanoparticle concentration or nanoparticle number decreases by a factor of more or less than 1.1, 2, 5, 10, $10^2$, $10^3$, $10^5$, or $10^{10}$. For example, a SAR of 1 W per gram of nanoparticles for $10^7$ nanoparticles can lead to a SAR value of $10^{-7}$ W per gram of nanoparticle for a single nanoparticle. This behavior may be explained by a collective effect in which the SAR values of the individual nanoparticles would add to yield a SAR for the assembly of the nanoparticles, which is the sum of or is proportional to the SAR value of a single nanoparticle.

In one embodiment of the invention, nanoparticle(s), or the nanoparticle(s), represent(s) an assembly or suspension of more or less than 1, 10, $10^2$, $10^3$, $10^5$, $10^{10}$, $10^{20}$, or $10^{50}$ nanoparticle(s).

In another embodiment of the invention, the nanoparticle region represents the volume comprising an assembly of more or less than 1, 10, $10^2$, $10^3$, $10^5$, $10^{10}$, $10^{20}$, or $10^{50}$ nanoparticle(s). The nanoparticle region can also be defined as the space, surface, or volume, where nanoparticles are located, preferentially comprising more or less than 1, 2, 5, 10, $10^2$, $10^3$, $10^5$, $10^{10}$, $10^{15}$, $10^{20}$, or $10^{50}$ nanoparticle(s).

In one embodiment of the invention, the nanoparticle is defined as a particle with a size in one dimension, which is: i), larger than $10^{-3}$, $10^{-2}$, $10^{-1}$, 1, 2, 5, 10, 20, 50, 70, 100, 200, or 500 nm, or ii), lower than $10^4$, $10^3$, $10^2$, 10, 1, or $10^{-1}$ nm, or iii), lies between $10^{-2}$ and $10^4$ nm, between $10^{-1}$ and $10^3$ nm, or between 1 and $10^2$ nm.

In another embodiment of the invention, the nanoparticle has a surface charge, which is: i), larger or lower than -200, -100, -50, -10, -5, 0.1, 1, 2, 5, 10, 50, or 100 mV, preferentially at a pH lower or larger than 0.1, 1, 2, 3, 4, 5, 6, 7, 8, 9, 10, 11, 12, 13, or 14, or ii), comprised between +200 and -200 mV, +100 and -100 mV, +50 and -50 mV, +40 et-40mV, +20 and - 20, +10 and -10 mV, or between +5 and -5 mV, preferentially at a pH lower or larger than 0.1, 1, 2, 3, 4, 5, 6, 7, 8, 9, 10, 11, 12, 13, or 14.

In another embodiment of the invention, the nanoparticle has a weight or a mass, preferentially expressed in unit such as gram (g), kilogram (kg), or milligram (mg). A gram of nanoparticle can be a gram of metal such as iron comprised in the nanoparticle.

In another embodiment of the invention, the nanoparticle or nanoparticle assembly or nanoparticle suspension has a concentration, preferentially expressed in unit such as gram (g), kilogram (kg), milligram (mg), per unit volume such as liter (1), milliliter (ml), $cm^3$, or $m^3$ or per unit surface or per unit length, preferentially before or after administration in the body part, wherein the volume, surface, or length, is preferentially the volume, surface, or length of the nanoparticle, nanoparticle suspension or nanoparticle assembly, before or after administration in the body part, or the volume, surface, or length of the body part.

In another embodiment of the invention, the nanoparticle is surrounded by an organic or inorganic coating, wherein the coating preferentially enables to bind two or more nanoparticles together preferentially in a chain and/or prevents nanoparticle aggregation and/or enables uniform nanoparticle distribution.

In another embodiment of the invention, the nanoparticles are arranged in chains, wherein the chains are characterized by at least one of the following properties: i), a length that is preferentially shorter or longer than $2.10^5$, $2.10^3$, or $2.10^2$ nm, ii), a number of nanoparticles in each chain preferentially lower or larger than 1, 2, 5, 10, $10^2$, or $10^3$, or iii), the alignment of crystallographic directions between two adjacent nanoparticles in the chain, wherein such alignment is preferentially characterized by an angle between two crystallographic directions belonging to two adjacent nanoparticles in the chains of more or less than 90, 80, 70, 60, 50, 20, 10, 3, 2 ° (degree).

In another embodiment of the invention, the nanoparticles are assimilated to or are comprised in a ferrofluid, preferentially a chemical or biological ferrofluid, wherein chemical and biological ferrofluids are fluids containing iron, preferentially forming nanoparticles, which are fabricated through a chemical and biological synthesis, respectively. A chemical synthesis can be defined as a synthesis involving a majority of steps, or more than 1, 2, 5, or 10 steps, or more than 1, 2, 5, 25, 50, 75, or 90% of steps, which involve chemical reactions occurring without the involvement of living organisms, or parts of living organisms such as cells, organelles, DNA, RNA, proteins, enzymes, lipids. A biological synthesis can be defined as a synthesis involving a majority of steps, or more than 1, 2, 5, or 10 step(s), or more than 1, 2, 5, 25, 50, 75, or 90% of steps, which involve chemical reactions occurring with the involvement of at least 1, 2, 10, $10^3$, $10^6$, or $10^9$ living organism(s), or parts of living organisms such as cells, organelles, DNA, RNA, proteins, enzymes, lipids. The ferrofluid can comprise the magnetosomes and an excipient, a solvent, a matrix, a gel, which preferentially enables the administration of the magnetosomes to the individual.

In one embodiment of the invention, the nanoparticle is a magnetosome. Magnetosomes are characterized by at least one of the following properties: i), the presence of a mineral magnetic core, preferentially composed of iron oxide, most preferentially maghemite or magnetite, or an intermediate composition between maghemite and magnetite, ii), the presence of a coating that surrounds the magnetic core and preferentially prevents nanoparticle aggregation, preferentially enabling nanoparticle administration in an organism or stabilizing the nanoparticle magnetic core, where coating thickness may preferably lie between 0.1 nm and 10 μm, 0.1 nm and 1 μm, 0.1 nm and 100 nm, 0.1 nm and 10 nm, or between 1 nm and 5 nm, iii), magnetic properties leading to diamagnetic, paramagnetic, superparamagnetic, ferromagnetic, or ferrimagnetic behavior, iv), magnetic properties associated to a coercivity larger or lower than 0.01, 0.1, 1, 10, 100, $10^3$, $10^4$, $10^5$, $10^9$, or $10^{20}$ Oe, v), magnetic properties associated to a ratio between remanent and saturating magnetization larger than 0.01, 0.1, 0.2, 0.3, 0.4, 0.5, 0.75, 0.9, or 0.99 vi), magnetic properties associated to a saturating magnetization larger than 0.1, 1, 5, 10, or 50 emu/g, vii), magnetic properties preferentially measured or observed at a temperature larger or lower than 0.1 K, 1 K, 10 K, 20 K, 50 K, 100 K, 200 K, 300 K, 350 K, or 3000 K, viii), a crystallinity, *i.e.* magnetosomes preferentially possessing at least 1, 2, 5, 10, or 100 crystalline plane(s), preferentially observable or measured by electron microscopy, ix), the presence of a single domain, x), a size that is are larger or smaller than 0.1, 0.5, 1.5, 10, 15, 20, 25, 30, 50, 60, 70, 80, 100, 120, 150, or 200 nm, xi), a size lying between 0.1 nm and 10 μm, 0.1 nm and 1 μm, 0.1 nm and 100 nm, 1 nm and 100 nm, or between 5 nm and 80 nm, xii), a non-pyrogenicity, *i.e.* magnetosomes possess an endotoxin concentration lower than 10000, 1000, 100, 50, 10, 5, 2, or 1 EU (endotoxin unit) per mg of nanoparticle or per mg of iron comprised in magnetosomes, xiii), a synthesis by a synthetizing living organism, preferentially by magnetotactic bacteria, leading to the production of magnetosomes, preferentially extracted from magnetotactic bacteria, preferentially only or mostly comprising the mineral magnetic core of the magnetosomes, xiv), the presence of less than 50, 25, 15, 10, 5, 2, or 1% of organic or carbon material originating from the synthetizing living organism, xv), the presence of more than 99, 95, 80, 70, 60, 50, or 25% of mineral magnetic material originating from the synthetizing living organism, xvi), a specific absorption rate (SAR) that is larger or lower than 1, 10, 1000, or $10^4$ Watt per gram of nanoparticle, preferentially under the application of an alternating magnetic field of strength preferentially larger or lower than 0.1, 1, 10, or 100 mT, and/or frequency larger or lower than 1, 10, 100, or 1000 KHz.

In one embodiment of the invention, magnetosomes are arranged in chains comprising more than 1, 2, 3, 4, 5, 6, 7, 8, 9, 10, 15, 20, 25, 30, 35, or 40 magnetosome(s). Magnetosomes can be arranged in chains inside magnetotactic bacteria or outside magnetotactic bacteria, preferentially after their extraction from magnetotactic bacteria.

In one embodiment of the invention, magnetosomes are not arranged in chains.

In another embodiment of the invention, magnetosomes are purified to remove more than 10, 50, or 90% of endotoxins and/or other biological material such as proteins and lipids originating from magnetotactic bacteria. Such purification can use detergent such as NaOH or KOH. Purified magnetosomes can be recoated with a synthetic coating such as a compound comprising a function selected in the group comprising carboxylic acids, phosphoric acids, sulfonic acids, esters, amides, ketones, alcohols, phenols, thiols, amines, ether, sulfides, acid anhydrides, acyl halides, amidines, amides, nitriles, hydroperoxides, imines, aldehydes, or peroxides. The coating can be made of carboxy-methyl-dextran, citric acid, phosphatidylcholine or its derivative (DOPC), or oleic acid. The coating can enable the dispersion of the magnetosomes in a matrix or solvent such as water. Purified magnetosomes (recoated or not) are preferentially non-pyrogenic. They can comprise less than $10^8$, $10^5$, $10^3$, or 10 EU (endotoxin unit) per $mm^3$ or mL of magnetosomes, of magnetosome suspension, or of body part. Purified magnetosomes (recoated or not) can be re-suspended in a liquid or redispersed in a matrix to lead to a homogenous dispersion or to a high stability. A magnetosome suspension is preferentially stable at a concentration larger than 1, 5, 10, 50, 100, 200, 500, or 1000 mg per mL of solvent, *i.e.* preferentially the optical density of this suspension, preferentially measured at 480 nm, does not decrease by more than 1, 5, 10, 50, 75, or 90 % within more or less than 1, 5, 10, $10^3$, $10^7$, or $10^{20}$ seconds.

In one embodiment of the invention, magnetosomes are synthesized by magnetotactic bacteria such as such as Magnetospirillum magneticum strain AMB-1, magnetotactic coccus strain MC-1, three facultative anaerobic vibrios strains MV-1, MV-2 and MV-4, the Magnetospirillum magnetotacticum strain MS-1, the Magnetospirillum gryphiswaldense strain MSR-1, a facultative anaerobic magnetotactic spirillum, Magnetospirillum magneticum strain MGT-1, and an obligate anaerobe, Desulfovibrio magneticus RS-1.

In one embodiment of the invention, a magnetotactic bacterium is defined as a bacterium able to synthesize nanoparticles, wherein these nanoparticles are preferentially characterized by at least one of the following properties: i), they are produced intracellularly, ii), they are magnetic, iii), they comprise a mineral, iv), their core is preferentially composed of a metallic oxide such as iron oxide, v), their core is surrounded by biological material such as lipids, proteins, endotoxins, which can preferentially be removed, v), they are arranged in chains, vi), they produce heat under the application of an alternating magnetic field.

In one embodiment of the invention, magnetosomes comprise the mineral part synthesized by magnetotactic bacteria, *i.e.* preferentially the crystallized iron oxide produced by these bacteria, and/or do not comprise proteins, lipids, endotoxins, or biological materials comprising carbon or more than 0.1, 1, 10, 30, 50, 75% of carbon, which is/are produced by these bacteria.

In one embodiment of the invention, chemical nanoparticles, *i.e.* nanoparticles that have not been synthesized by the synthetizing living organism, most preferentially by a magnetotactic bacterium, can possess at least 1, 2, 3, 4, 5, 6, 7, 8, 9, or 10 common property(ies) with the magnetosome.

In one embodiment of the invention, the specific absorption rate (SAR) of the nanoparticles, designated as $SAR_{real}$, is the specific absorption rate of the nanoparticles comprised, mixed or inserted in a body part, designated as $SAR_{(N)}$, minus the specific absorption rate of the body part without the nanoparticles, designated as $SAR_{(WN)}$. It can be expressed using the formula $SAR_{real}=SAR_{(N)}-SAR_{(WN)}$. It can be expressed in a power unit such as Watt divided by a mass unit such as gram or in a power unit divided by a length, surface, or volume unit such as cm, $cm^2$, or $cm^3$. $SAR_{(N)}$ and $SAR_{(WN)}$ are preferentially measured under the application of a radiation that produces a temperature increase, such as an acoustic wave, preferentially of power density larger than $10^{-9}$, $10^{-5}$, $10^{-3}$, $10^{-1}$, 1, 10, or $10^3$ W/cm, $W/cm^2$, or $W/cm^3$, preferentially of frequency larger than $10^{-6}$, $10^{-3}$, $10^{-1}$, 1, 10, $10^3$, or $10^6$ MHz, an alternating magnetic field, preferentially of frequency larger than $10^{-9}$, $10^{-6}$, $10^{-3}$, 1, $10^3$, $10^6$, or $10^9$ kHz, preferentially of strength larger than $10^{-9}$, $10^{-6}$, $10^{-3}$, $10^{-1}$, 1, 10, $10^3$, or $10^6$ mT, or a laser, preferentially of power density larger than $10^{-9}$, $10^{-5}$, $10^{-3}$, $10^{-1}$, 1, 10, or $10^3$ W/cm, $W/cm^2$, or $W/cm^3$. $SAR_{(N)}$ and $SAR_{(WN)}$ are preferentially measured in adiabatic conditions or in conditions in which heat exchanges are minimized, preferentially between: i), the body part comprising the nanoparticles and the region outside the body part comprising the nanoparticles, or ii), the container or tube containing the nanoparticles and the exterior of this container or tube. Heat exchanges are minimized when they produce a temperature decrease of less than 75, 60, 50, 25, 10, 5, 2, 1, or 0.1 °C. In some cases, $SAR_{(WN)}$ is proportional to the initial slope of the temperature variation with time of the body part, medium, water, tissue without the nanoparticles, $(\Delta T/\delta t)_{(WN)}$, preferentially estimated in °C/sec, preferentially leading to the formula: $SAR_{(WN)}=\alpha_{WN}.(\Delta T/\delta t)_{(WN)}$, where $\alpha_{WN}$ is a proportionality coefficient. In some cases, $SAR_{(N)}$ is proportional to the initial slope of the temperature variation with time of the nanoparticles comprised in the body part, $(\Delta T/\delta t)_{(N)}$, preferentially estimated in °C/sec, preferentially leading to the formula: $SAR_{(N)}=\alpha_N.(\Delta T/\delta t)_{(N)}$, where $\alpha_N$ is a proportionality coefficient. In some cases, $SAR_{rea}=[(\Delta T/\delta t)_{(N)}-(\Delta T/\delta t)_{(WN)}].C_v/C_{nano}$, where $C_v$ is the specific heat capacity, preferentially of the body part, tissue, water, medium comprising the nanoparticles, and $C_{nano}$ is the nanoparticle concentration, preferentially comprised in the body part.

In one embodiment of the invention, the initial slope of the temperature variation with time of the nanoparticles, $(\Delta T/\delta t)$, designated as $(\Delta T/\delta t)_{real}$, is the initial slope of the temperature variation with time of the nanoparticles comprised, mixed or inserted in a body part, designated as $(\Delta T/\delta t)_{(N)}$, minus the initial slope of the temperature variation with time of the body part without the nanoparticles, designated as $(\Delta T/\delta t)_{(WN)}$, $(\Delta T/\delta t)_{real} = (\Delta T/\delta t)_{(N)} - (\Delta T/\delta t)_{(WN)}$. These initial slopes can be the slopes of the temperature variation with time measured: i), during the first $10^{-9}$, $10^{-6}$, $10^{-3}$, $10^{-1}$, 1, 10, $10^3$, or $10^5$ minute(s) of the application of the acoustic wave, ii), when the temperature varies linearly with time, iii), before the saturating temperature has been reached, iv), during an initial time of the heating step, which represents less than 1, 5, 10, 25, 50, 75, 80, 90, 95, or 99% of the total duration of the heating step.

In another embodiment of the invention, the values of $SAR_{(N)}$, $SAR_{(WN)}$ and/or $SAR_{real}$ are larger or lower than $10^{-9}$, $10^{-6}$, $10^{-3}$, 1, $10^3$, $10^6$, or $10^9$ Watt per gram of nanoparticles ($W/g_{nano}$) or $10^9$ Watt per $cm^3$ of nanoparticles ($W/cm^3_{nano}$) or $10^9$ Watt per gram of body part ($W/g_{bp}$) or $10^9$ Watt per $cm^3$ of body part ($W/cm^3_{bp}$).

In still another embodiment of the invention, the values of $\alpha_N$ and/or $\alpha_{WN}$ are smaller or larger than $10^{-9}$, $10^{-6}$, $10^{-3}$, 1, $10^3$, $10^6$, or $10^9$ (sec/°C).($W/g_{nano}$) or $10^9$ (sec/°C).($W/g_{bp}$) or $10^9$ (sec/°C).($W/cm^3_{bp}$) or $10^9$ (sec/°C).($W/cm^3_{nao}$).

In one embodiment of the invention, an acoustic wave medical treatment is a medical treatment also designated as treatment, which uses acoustic wave energy, preferentially applied on nanoparticles or body part, to trigger a medical, pharmaceutical, immunological, metabolic, diagnostic, medical device, drug, biological, or cosmetic effect. In some cases, a medical treatment can be the treatment of an illness such as an infectious disease, a cancer, or a therapeutic treatment. It can be the treatment of a disease due to the malfunction of an organ or body part. It can be due to the malfunction of a body part of an individual. In some cases, a medical treatment can be a diagnostic of a disease or a cosmetic treatment.

In an embodiment of the invention, the organ or body part belongs to the musculoskeletal, muscular, digestive, respiratory, urinary, female reproductive, male reproductive, circulatory, cardiovascular, endocrine, circulatory, lymphatic, nervous (peripheral or not), ventricular, enteric nervous, sensory, or integumentary system, reproductive organ (internal or external), sensory organ, endocrine glands. The organ or body part can be human skeleton, joints, ligaments, tendons, mouth, teeth, tongue, salivary glands, parotid glands, submandibular glands, sublingual glands, pharynx, esophagus, stomach, small intestine, duodenum, jejunum, ileum, large intestine, liver, gallbladder, mesentery, pancreas, nasal cavity, pharynx, larynx, trachea, bronchi, lungs, diaphragm, kidneys, ureters, bladder, urethra, ovaries, fallopian tubes, uterus, vagina, vulva, clitoris, placenta, testes, epididymis, vas deferens, seminal vesicles, prostate, bulbourethral glands, penis, scrotum, pituitary gland, pineal gland, thyroid gland, parathyroid glands, adrenal glands, pancreas, heart, arteries, veins, capillaries, lymphatic vessel, lymph node, bone marrow, thymus, spleen, gut-associated lymphoid tissue, tonsils, brain, cerebrum, cerebral hemispheres, diencephalon, brainstem, midbrain, pons, medulla, oblongata, cerebellum, spinal cord, choroid plexus, nerves, cranial nerves, spinal nerves, ganglia, eye, cornea, iris, ciliary body, lens, retina, ear, outer ear, earlobe, eardrum, middle ear, ossicles, inner ear, cochlea, vestibule of the ear, semicircular canals, olfactory epithelium, tongue, taste buds, mammary glands, or Skin. The body part or organ can belong to the blood circulation.

In an embodiment of the invention, the nanoparticles are drugs, medical devices, cosmetic products, or products used to determine the properties of biological samples.

In one embodiment of the invention, an acoustic wave medical treatment is a treatment, which induces the death, destruction, denaturation, or inactivation of at least 1, 10, $10^3$, $10^6$, or $10^9$ biological material(s), such as cell(s), preferentially pathological cell(s), RNA, DNA, protein(s), lipid(s), or enzyme(s). The death of cell(s) can occur through apoptosis or necrosis, but is preferentially occurring through apoptosis.

The body part of an individual also designated as body part can represent part of an individual, where the individual is preferentially a human, an animal, or an organism comprising at least one prokaryotic or eukaryotic cell, or an assembly of at least one prokaryotic or eukaryotic cell.

In one embodiment of the invention, the body part is alive (or not), is any tissue, water, medium, substance, cell, organelle, organ protein, lipid, DNA, RNA, biological material, preferentially localized in a specific region of an individual, preferentially originating or extracted from such region.

In one embodiment of the invention, the nanoparticles are administered to or in the body part, when they are directly administered in or to the body part, preferentially less than 1, $10^{-1}$, $10^{-2}$, $10^{-3}$, $10^{-4}$, $10^{-5}$, $10^{-6}$, or $10^{-9}$ m away from the body part.

In another embodiment of the invention, the nanoparticles are administered to or in the body part, when they are administered far from the body part, preferentially more than 1, $10^{-1}$, $10^{-2}$, $10^{-3}$, $10^{-4}$, $10^{-5}$, $10^{-6}$, or $10^{-9}$ m away from the body part.

In another embodiment of the invention, the nanoparticles are administered to or in the body part following at least one of the following administration routes: local, enteral, gastrointestinal, parenteral, topical, oral, inhalation, intramuscular, subcutaneous, intra-tumor, in an organ, in a vein, in arteries, in blood, or in tissue.

In one embodiment of the invention, the body part is a pathological site. The pathological site can be defined as an unhealthy site, or a site that is in a different condition from a site of a healthy individual, or the site of an unhealthy individual. It can comprise pathological cells, such as tumor cells, bacteria, eukaryotic or prokaryotic cells, viruses or other pathological material. It can also comprise healthy cells, which are not arranged or working as they usual do in a healthy individual. For example, it can comprise a larger or lower number of healthy cells, or it can comprise healthy cells that do not function as they usually do in a healthy individual.

In another embodiment of the invention, the body part, healthy or pathological site, or nanoparticle region, has a size, which is larger or lower than 1, $10^{-1}$, $10^{-2}$, $10^{-3}$, $10^{-4}$, $10^{-5}$, $10^{-6}$, $10^{-7}$, $10^{-8}$, or $10^{-9}$ m.

In another embodiment of the invention, the pathological site is defined as a site or region that comprises more or less than 1, 10, $10^3$, $10^5$, $10^7$, $10^9$, or $10^{20}$ pathological cell(s). A pathological cell can be defined as a cell that does not work as a healthy cell, for example a cell that divides more quickly than a healthy cell.

In another embodiment of the invention, the healthy site is defined as a site or region that comprises more or less than 1, 10, $10^3$, $10^5$, $10^7$, $10^9$, or $10^{20}$ healthy cell(s). A healthy cell can be defined as a cell that belongs to a healthy individual or to the body part of a healthy individual.

In some cases, the body part can comprise the pathological site, the healthy site, or the nanoparticle region. The body part can comprise: i), more or less than $10^{-9}$, $10^{-7}$, $10^{-5}$, $10^{-3}$, $10^{-1}$, 1, 10, $10^3$, $10^5$, $10^7$, or $10^9$ mg of nanoparticles, preferentially per $mm^3$ or per $cm^3$ of body part, or ii), more or less than $10^{-9}$, $10^{-7}$, $10^{-5}$, $10^{-3}$, $10^{-1}$, 1, 10, $10^3$, $10^5$, $10^7$, or $10^9$ pathological or healthy cells, preferentially per $mm^3$ or per $cm^3$ of body part.

In some cases, the body part can be associated to: i), the body part exposed (or not) to the acoustic wave(s), or ii), the body part receiving (or not) the acoustic wave energy.

The invention also relates to nanoparticles for use in an acoustic wave medical treatment of a body part of an individual, wherein the nanoparticles are administered to the body part and produce a slope of the initial variation of temperature with time, which is larger than $10^{-9}$ °C per second per gram of nanoparticle.

In one embodiment of the invention, the values of $(\Delta T/\delta t)_{(N)}$, $(\Delta T/\delta t)_{(WN)}$, and/or $(\Delta T/\delta t)_{real}$, are larger or lower than $10^{-9}$, $10^{-6}$, $10^{-3}$, $10^{-1}$, 1, $10^3$, $10^6$, or $10^9$ °C/sec.

In another embodiment of the invention, the values of $(\Delta T/\delta t)_{(N)}$, $(\Delta T/\delta t)_{(WN)}$ and/or $(\Delta T/\delta t)_{(real)}$ are larger or lower than $10^{-9}$, $10^{-6}$, $10^{-3}$, $10^{-1}$, 1, $10^3$, $10^6$, or $10^9$ °C/sec per gram of nanoparticle or $10^9$ °C/sec per gram of body part or $10^9$ °C/sec per $cm^3$ of nanoparticle, per $cm^2$ of nanoparticle, per cm of nanoparticle or $10^9$ °C/sec per $cm^3$ of body part, per $cm^2$ of body part, or per cm of body part.

The invention also relates to nanoparticles for use, wherein the medical treatment is the treatment of an infectious disease, an auto-immune disease, a neuropathology, a cancer, a cutaneous condition, an endocrine disease or disorder, an intestinal disease, a communication disorder, a genetic disorder, a neurological disorder, a voice disorder, a vulvovaginal disorder, a liver disorder, a heart disorder, a heating disorder, a mood disorder, or a personality disorder.

The invention also relates to nanoparticles for use, wherein the nanoparticles are crystallized, metallic, or magnetic.

In an embodiment of the invention, the nanoparticles are crystallized. In this case, they preferentially possess more than 1, 2, 10, $10^2$, $10^3$, $10^6$, or $10^9$ crystallographic plane(s) or regular atomic arrangement(s), preferentially observable by electron microscopy.

In another embodiment of the invention, the nanoparticles are metallic. In this case, they preferentially comprise at least 1, 10, $10^3$, $10^5$, or $10^9$ metallic atom(s). In some cases, they can also comprise more than 1, 10, $10^3$, $10^5$, or $10^9$ oxygen atom(s).

In one embodiment of the invention, the nanoparticles are magnetic. In this case, they can have a diamagnetic, superparamagnetic, paramagnetic, ferromagnetic, or ferrimagnetic behavior, preferentially at a temperature, which is lower or larger than 0.5, 1, 10, 20, 50, 100, 200, 350, 500, $10^3$, or $10^5$ K.

The invention also relates to nanoparticles for use, wherein the thermal conductivity or density of the body part, the velocity of the acoustic wave, attenuation of the acoustic wave, absorption of the acoustic wave, elasticity of the acoustic wave, or acoustic impedance of the acoustic wave, is at least 1.1 larger or lower in the body part comprising the nanoparticles than in the body part without the nanoparticles.

In one embodiment of the invention, the thermal conductivity or density of the body part, the velocity of the acoustic wave, attenuation of the acoustic wave, absorption of the acoustic wave, elasticity of the acoustic wave, or acoustic impedance of the acoustic wave, is at least 1.001, 1.01, 1.1, 1.2, 1.5, 2, 5, 10, 50, $10^2$, $10^3$, or $10^5$ larger or lower in the body part comprising the nanoparticles than in the body part without the nanoparticles.

In some cases, the thermal conductivity of the body part is at least $10^{20}$, $10^{10}$, $10^5$, $10^3$, $10^2$, 50, 10, 5, 2, 1, $10^{-1}$, $10^{-2}$, $10^{-3}$, $10^{-5}$, $10^{-10}$, or $10^{-20}$ W/m.K (Watt/meter.kelvin) larger or lower in the body part comprising the nanoparticles than in the body part without the nanoparticles.

In some case, the density of the body part is at least $10^{-9}$, $10^{-6}$, $10^{-3}$, $10^{-1}$, 1, 2, 5, 10, $10^3$, or $10^6$ $g/cm^3$ larger or lower in the body part comprising the nanoparticles than in the body part without the nanoparticles.

In some cases, the velocity of the acoustic wave is at least $10^{-5}$, $10^{-3}$, $10^{-1}$, 1, 10, 100, 1000, 1500, 2000, 3000, 5000, $10^4$, or $10^6$ m/s larger or lower in the body part comprising the nanoparticles than in the body part without the nanoparticles.

In some cases, the attenuation of the acoustic wave in the body part comprising the nanoparticles is at least $10^{-5}$, $10^{-3}$, $10^{-1}$, 1, 10, 100, $10^3$, or $10^5$ dB/cm larger or lower in the body part comprising the nanoparticles than in the body part without the nanoparticles.

In some cases, the acoustic impedance of the acoustic wave in the body part comprising the nanoparticles is at least $10^{-3}$, $10^{-2}$, $10^{-1}$, 0.5, 1, 1.5, 2, 5, 10, $10^2$, $10^4$, $10^6$, $10^9$, or $10^{20}$ MRayl or $Kg.m^{-2}s^{-1}$ larger or lower in the body part comprising the nanoparticles than in the body part without the nanoparticles.

In another embodiment of the invention, the increase, decrease, or variation of thermal conductivity or density of the body part, the velocity of the acoustic wave, attenuation of the acoustic wave, absorption of the acoustic wave, elasticity of the acoustic wave, or acoustic impedance of the acoustic wave, between the body part without the nanoparticles and the body part with the nanoparticles, is due to at least one of the following properties: i), a nanoparticle concentration in the body part that is larger or lower than $10^{-9}$, $10^{-7}$, $10^{-5}$, $10^{-3}$, $10^{-1}$, 1, 10, $10^{3}$, $10^{5}$, $10^{7}$, or $10^{9}$ mg per $mm^{3}$ or per $cm^{3}$ of body part, ii), a nanoparticle size that is larger or lower than $10^{-6}$, $10^{-3}$, $10^{-1}$, 1, $10^{3}$, or $10^{6}$ nm, iii), a nanoparticle arrangement in chains, or iv), nanoparticles forming aggregates.

This invention also relates to nanoparticles for use, wherein the nanoparticles are administered to the body part and the body part is sequentially exposed to acoustic waves of intensity lower than 1000 $W/cm^{2}$. In some cases, the intensity of the acoustic wave can be lower than 500, 10, 5, or 1 $W/cm^{2}$.

In one embodiment of the invention, the acoustic wave can induce a movement or vibration of the nanoparticle. This is preferentially the case when the mass of the nanoparticles is lower than $10^{20}$, $10^{10}$, $10^{5}$, $10^{3}$, $10^{2}$, 1, $10^{-3}$, or $10^{-5}$ $\mu$g per nanoparticle. In this case, the acoustic wave can induce either a larger or lower movement or vibration for the nanoparticles than for the other substances, atoms, ions, which are not arranged or assembled in nanoparticles, and preferentially surround the nanoparticles.

In another embodiment of the invention, the acoustic wave can be absorbed by the nanoparticles. In this case, the acoustic wave energy can either be absorbed more or less importantly by the nanoparticle than by the other substances, atoms, ions, which are not arranged or assembled in nanoparticles, and preferentially surround the nanoparticles.

In one embodiment of the invention, the acoustic wave intensity, power, energy, or frequency is the acoustic wave intensity or frequency generated by an apparatus generating acoustic waves, or by a transducer. It can be the acoustic wave intensity, power, energy, or frequency measured just after the acoustic wave has left the apparatus generating the acoustic wave or transducer. It can also be the acoustic wave intensity, power, energy, or frequency measured after the acoustic wave has left the apparatus generating the acoustic wave or transducer and travelled through another medium (liquid, gaz, solid), such as the body part.

In one embodiment of the invention, the acoustic wave is applied sequentially. A sequence can correspond to or be the application of the acoustic wave during a time $t_1$ followed by the non-application of the acoustic wave during a time $t_2$. A sequence can also correspond to or be the application of an acoustic wave during a time $t_1$ followed by the application of another acoustic wave during a time $t_3$, wherein the intensity, power, energy, or frequency of the acoustic wave applied during the time $t_3$ is lower than the intensity, power, energy, or frequency of the acoustic wave applied during the time $t_1$.

In one embodiment of the invention, the time $t_1$ is the duration of a heating step.

In another embodiment of the invention, the time $t_2$ or $t_3$ is the duration of a cooling step.

In one embodiment of the invention, the time $t_1$, $t_2$, or $t_3$, is preferentially longer or shorter than $10^{-3}$, $10^{-2}$, $10^{-1}$, 1, 10, $10^{2}$, or $10^{3}$ minute(s). In some cases, $t_1$, $t_2$, or $t_3$, is preferentially longer or shorter than $10^{-9}$, $10^{-7}$, $10^{-5}$, $10^{-3}$, $10^{-1}$, 1, 10, $10^{3}$, $10^{5}$, $10^{7}$, or $10^{9}$ seconds.

In one embodiment of the invention, the time $t_1$, $t_2$, or $t_3$, is preferentially longer or shorter than the time of a pulse, preferentially by a factor of at least 1.1, 1.5, 2, 5, 10, $10^{2}$, $10^{3}$, $10^{5}$, $10^{7}$, $10^{9}$, $10^{12}$, $10^{15}$, or $10^{20}$.

In one embodiment of the invention, the ratio $t_1/t_2$ or $t_2/t_3$ is larger or smaller than $10^{-9}$, $10^{-6}$, $10^{-3}$, $10^{-1}$, 1, 10, $10^{3}$, $10^{6}$, or $10^{9}$.

In one embodiment of the invention, the acoustic wave intensity, energy, power, or frequency applied during $t_3$ is at least 1.1, 1.3, 1.5, 2, 5, 10, $10^{2}$, $10^{3}$, $10^{5}$, $10^{7}$, or $10^{9}$ lower than the acoustic wave intensity, energy, power, or frequency applied during $t_1$.

In one embodiment of the invention, the acoustic wave intensity applied during $t_1$ or $t_3$ is lower than $10^{9}$, $10^{6}$, $10^{3}$, 100, 10, 1, $10^{-1}$, $10^{-2}$, or $10^{-5}$ $W/cm^{2}$.

In one embodiment of the invention, the acoustic wave power applied during $t_1$ or $t_3$ is lower than $10^{9}$, $10^{6}$, $10^{3}$, 100, 10, 1, $10^{-1}$, $10^{-2}$, or $10^{-5}$ W.

In one embodiment of the invention, the acoustic wave power density applied during $t_1$ or $t_3$ is lower than $10^{9}$, $10^{6}$, $10^{3}$, 100, 10, 1, $10^{-1}$, $10^{-2}$, or $10^{-5}$ W/cm, or $10^{-5}$ $W/cm^{2}$, or $10^{-5}$ $W/cm^{3}$. In one embodiment of the invention, the acoustic wave energy or energy density applied during $t_1$ or $t_3$ is lower than $10^{9}$, $10^{6}$, $10^{3}$, 100, 10, 1, $10^{-1}$, $10^{-2}$, or $10^{-5}$ W.sec, or $10^{-5}$ W.sec/cm, or $10^{-5}$ W.sec/$cm^{2}$, or $10^{-5}$ W.sec/$cm^{3}$.

In one embodiment of the invention, the acoustic wave frequency applied during $t_1$ or $t_3$ is lower than $10^{3}$, 10, 1, $10^{-3}$, $10^{-6}$, $10^{-9}$, or $10^{-20}$ GHz.

In one embodiment of the invention, a sequence, preferentially of duration $t_1+t_2$ or $t_1+t_3$, is repeated more than 1, 2, 3, 4, 5, 6, 7, 8, 9, 10, 250, 500, $10^{2}$, $10^{3}$, $10^{5}$, $10^{7}$, or $10^{9}$ time(s). In some cases, the treatment can comprise more or less than 1, 2, 3, 4, 5, 6, 7, 8, 9, 10, 250, 500, $10^{2}$, $10^{3}$, $10^{5}$, $10^{7}$, or $10^{9}$ sequence(s).

In another embodiment of the invention, the nanoparticles remain in the body part during the treatment, preferentially during more or less than 1, 2, 5, 10, 20, 50, 100, $10^{3}$, or $10^{4}$ sequence(s), preferentially during more or less than 1, 2, 5, 10, 50, 100, or $10^{3}$ day(s). In some cases, the nanoparticles remain in the body part during the treatment without decreasing in size by more than 1, 10, 20, 50, 100, 500, $10^{3}$, or $10^{4}$% compared with the size of the nanoparticles before administration in the body part or before the beginning of the treatment. In one embodiment of the invention, the

times $t_1$ and $t_2$ or the times $t_1$ and $t_3$ do not vary by more than $10^9$, $10^5$, $10^3$, 10, or $10^{-1}$% between two sequences. In some cases, this percentage of variation can be estimated as the absolute value of $(t_1-t_2)/t_1$ or as the absolute value of $(t_1-t_3)/t_1$.

In one embodiment of the invention, the time $t_1$, $t_2$, or $t_3$ is chosen to reach a desired parameter, which preferentially makes the treatment efficient. For example, it is chosen to reach: i), a desired temperature, ii), a desired level of cavitation, iii), a desired level or concentration of free radicals or radical oxygen species (ROS), iv), a desired level of apoptosis or desired number of apoptotic cells. In this case, the desired temperature, or desired level of cavitation, or desired level or concentration of free radicals or ROS, or desired level of apoptosis or desired number of apoptotic cells, are preferentially higher or larger during the time $t_1$ than during $t_2$ or $t_3$ by a factor, which is preferentially at least equal to 1, 1.2, 1.5, 2, 5, 10, $10^2$, $10^3$, $10^5$, $10^9$, or $10^{20}$.

In this invention, the temperature can be the temperature measured at the macroscopic scale, *i.e.* the temperature preferentially measured at a scale that comprises more than 1, 10, $10^3$, $10^5$, or $10^9$ nanoparticle(s) or at a scale larger than the size of 1, 10, $10^3$, $10^5$, or $10^9$ nanoparticle(s). In some cases, the temperature can be the temperature measured at the nanoscopic scale, *i.e.* the temperature preferentially measured at a scale that comprises less than 2, 5, 10, $10^3$, $10^5$, or $10^9$ nanoparticles or at a scale smaller than the size of 1, 2, 5, 10, $10^3$, $10^5$, or $10^9$ nanoparticle(s).

In another embodiment of the invention, the desired temperature is a temperature, which is preferentially larger than the physiological temperature by at least $10^{-5}$, $10^{-3}$, $10^{-1}$, 1, 2, 3, 4, 5, 7, 10, 15, 20, 25, 30, 50, 100, 200, 500, 1000, or 5000 °C. The desired level of cavitation is preferentially the desired number, size, or speed of bubbles, or to the desired temperature resulting from cavitation, that one wishes to reach during treatment. The desired number of bubbles can be larger or lower than 1, 2, 5, 10, $10^3$, $10^6$, $10^9$, or $10^{20}$ bubble(s) per nanoparticle or per assembly of nanoparticles. The desired size of bubbles can be larger or lower than $10^{-3}$, 1, 10, $10^2$, $10^3$, $10^5$, $10^{10}$, or $10^{20}$ nm. The desired speed of cavitation can be larger or lower than $10^{-3}$, 1, $10^2$, $10^3$, $10^5$, $10^7$, or $10^9$ m/sec. The desired temperature resulting from cavitation can be $10^{-5}$, $10^{-3}$, $10^{-1}$, 1, 2, 3, 4, 5, 7, 10, 15, 20, 25, 30, $10^2$, $10^3$, or $10^5$ °C above or below physiological temperature. The desired level of free radicals or ROS is preferentially the free radical or ROS concentration, which preferentially is, corresponds to, or leads to a $H_2O_2$ or free radical concentration, which is lower or larger than $10^{20}$, $10^{10}$, $10^5$, $10^3$, 10, 1, $10^{-3}$, or $10^{-5}$ nM of $H_2O_2$ or free radical per nanoparticles or per assembly of nanoparticles or per $cm^3$ or $mm^3$ of body part. The desired level of apoptosis is preferentially a number of apoptotic cells, which is lower or larger than 1, 5, 10, $10^2$, $10^3$, $10^6$, $10^9$, or $10^{20}$ or $10^{20}$ cell(s) per $mm^3$ or $cm^3$ of body part. The desired level of apoptotic cells can also be more or less than $10^{-9}$, $10^{-5}$, $10^{-2}$, 1, 5, 10, $10^3$, $10^5$, or $10^9$ % of the total number of pathological cells preferentially comprised in the body part.

In one embodiment of the invention, the desired temperature, level of cavitation, level of free radicals or ROS, or level of apoptosis, is the temperature, level of cavitation, level of free radicals of ROS, or level of apoptosis, reached during treatment, or reached during the time $t_1$, $t_2$, or $t_3$. It can also be a temperature, level of cavitation, level of free radicals of ROS, or level of apoptosis that does(do) not differ by more or less than $10^{-9}$, $10^{-5}$, $10^{-2}$, 1, 5, 10, $10^3$, $10^5$, or $10^9$ % from the desired temperature, desired level of cavitation, desired level of free radicals of ROS, or desired level of apoptosis.

In one embodiment of the invention, cavitation leads to the formation or nucleation of bubbles originating from the nanoparticles. In this case, the size or number of bubbles can differ by a factor of more or less than 1.1, 1.2, 1.5, 2, 2.5, 5, 10, $10^2$, $10^3$, or $10^5$, from the size or number of bubbles, which are not originating from the nanoparticles or are not produced by the nanoparticles or are produced in the absence of nanoparticles.

In one embodiment of the invention, heat or ROS produced by the acoustic wave, preferentially by the application of the acoustic waves on nanoparticles, is preferentially resulting from the implosion or destruction of cavitation bubbles, also preferentially designated as bubbles. In some cases, stable cavitation, which preferentially does not result in implosion or destruction of cavitation bubbles, can be distinguished from inertial cavitation, which preferentially results in implosion or destruction of cavitation bubbles.

In an embodiment of the invention, the acoustic wave has a low energy. In this case, the energy or energy density of the acoustic wave is preferentially lower than $10^9$, $10^6$, $10^3$, 100, 10, 1, $10^{-1}$, $10^{-2}$, or $10^{-5}$ W.sec, or $10^{-5}$ W.sec/cm, or $10^{-5}$ W.sec/$cm^2$, or $10^{-5}$ W.sec/$cm^3$. The acoustic wave can have a low energy when the power of the acoustic wave is low and/or when the time of application of the acoustic is short, preferentially shorter than 24, 12, 6, 3, 2, or 1 hour, or shorter than 50, 30, 15, 10, 5, 2, or 1 minute(s), or shorter than 50, 30, 20, 10, 5, 2, 1, 10-1, $10^{-3}$, $10^{-6}$, or $10^{-9}$ second(s).

In one embodiment of the invention, the acoustic wave has a low power. In this case, the power of the acoustic wave is preferentially lower than $10^9$, $10^6$, $10^3$, 100, 10, 1, $10^{-1}$, $10^{-2}$, or $10^{-5}$ W.

In one embodiment of the invention, the acoustic wave has a low power density. In this case, the power density of the acoustic wave is preferentially lower than $10^9$, $10^6$, $10^3$, 100, 10, 1, $10^{-1}$, $10^{-2}$, or $10^{-5}$ W/cm, or $10^{-5}$ W/$cm^2$, or $10^{-5}$ W/$cm^3$.

The invention also relates to the use of acoustic wave of low intensity. In this case, the acoustic wave intensity is preferentially lower than 100, 10, or 1 W/$cm^2$. The acoustic wave intensity can be lower than $10^9$, $10^5$, 100, 10, 1,

$10^{-1}$, $10^{-2}$, $10^{-1}$, $10^{-5}$, or $10^{-7}$ W/cm$^2$.

In one embodiment of the invention, the acoustic wave, preferentially of low intensity, energy, or power, applies a pressure, preferentially on nanoparticles or on the body part, which is larger or smaller than $10^{-9}$, $10^{-5}$, $10^{-3}$, $10^{-1}$, 1, 10, $10^3$, $10^6$, or $10^9$ MPa.

In one embodiment of the invention, nanoparticles are sonosensitizers, preferentially used in sonodynamic therapy.

In one embodiment of the invention, a sonosensitizer is defined as a substance that enhances the effect of acoustic waves, *i.e.* that increases the number of biological material(s), preferentially pathological cell(s), which is(are) dead, destroyed, denatured, or inactivated, by a factor of 1.2, 1.5, 2, 5, 10, $10^3$, or $10^5$ and/or that decreases the number of healthy cell(s), which is(are) dead, destroyed, denatured, or inactivated, by a factor of 1.2, 1.5, 2, 5, 10, $10^3$, or $10^5$, preferentially during medical treatment.

In one embodiment of the invention, sonodynamic therapy is defined as a therapy, which uses low intensity acoustic wave.

The invention also relates to nanoparticles for use, wherein the acoustic wave is an ultrasound.

The invention also relates to nanoparticles for use, wherein the frequency of the acoustic wave is lower than 100 MHz. In some cases, the acoustic wave frequency can be lower than $10^5$, $10^3$, $10^2$, 10, 1, $10^{-1}$, $10^{-2}$, $10^{-3}$, $10^{-4}$, $10^{-5}$, $10^{-6}$, $10^{-7}$, $10^{-8}$, $10^{-10}$, or $10^{-15}$ MHz.

The invention also relates to the use of an acoustic wave frequency, which is larger than $10^5$, $10^3$, $10^2$, 10, 1, $10^{-1}$, $10^{-2}$, $10^{-3}$, $10^{-4}$, $10^{-5}$, $10^{-6}$, $10^{-7}$, $10^{-8}$, $10^{-10}$, or $10^{-15}$ MHz.

In one embodiment of the invention, the acoustic wave frequency, intensity, energy, or power, is sufficiently low or large to enable internalization of the nanoparticles in cells preferentially belonging to the body part. It can be sufficiently low or large to enable the internalization of more or less than 1, 10, $10^3$, $10^6$, $10^9$, or $10^{20}$ nanoparticle(s) per cell, or $10^{-6}$, $10^{-3}$, $10^{-1}$, 1, 10, $10^3$, $10^6$, $10^9$, or $10^{20}$ nanoparticle(s) per cell or $10^{20}$ nanoparticles per mm$^3$ of body part or $10^{20}$ mg of nanoparticles per mm$^3$ of body part.

In one embodiment of the invention, the acoustic wave frequency, intensity, energy, or power, is sufficiently low or large to enable the destruction or growth inhibition of healthy or pathological cells preferentially belonging to the body part. It can be sufficiently low or large to enable the destruction or growth inhibition of more than 1, 10, $10^3$, $10^6$, $10^9$, or $10^{20}$ cell(s), preferentially using more or less $10^{-6}$, $10^{-3}$, $10^{-1}$, 1, 10, $10^3$, $10^6$, $10^9$, or $10^{20}$ nanoparticle(s) per cell or $10^{20}$ nanoparticles per mm$^3$ of body part or $10^{20}$ mg of nanoparticles per mm$^3$ of body part.

In one embodiment of the invention, the acoustic wave frequency, intensity, energy, or power, is sufficiently low or large to prevent internalization of the nanoparticles in healthy or pathological cells preferentially belonging to the body part. It can be sufficiently low or large to prevent the internalization of more or less than $10^{-6}$, $10^{-3}$, $10^{-1}$, 1, 10, $10^3$, $10^6$, $10^9$, or $10^{20}$ nanoparticle(s) per cell or $10^{20}$ nanoparticles per mm$^3$ of body part or $10^{20}$ mg of nanoparticles per mm$^3$ of body part.

In one embodiment of the invention, the acoustic wave frequency, intensity, energy, or power, is sufficiently low or large to prevent the destruction or growth inhibition of healthy or pathological cells preferentially belonging to the body part. It can be sufficiently low or large to prevent the destruction or growth inhibition of more than 1, 10, $10^3$, $10^6$, $10^9$, or $10^{20}$ cell(s), preferentially using more or less than $10^{-6}$, $10^{-3}$, $10^{-1}$, 1, 10, $10^3$, $10^6$, $10^9$, $10^{15}$, or $10^{20}$ nanoparticle(s) per cell or $10^{20}$ nanoparticle(s) per mm$^3$ of body part or $10^{20}$ mg of nanoparticles per mm$^3$ of body part.

In one embodiment of the invention, the acoustic wave frequency is non-thermal. In this case, the acoustic wave frequency is such that it can't induce an increase in temperature or it can induce an increase in temperature of less than $10^3$, $10^2$, 10, 5, 2, 1, or 0.1 °C, wherein the increase in temperature is preferentially occurring (or not) or measured (or not) in the body part without the nanoparticles, or in the nanoparticle region, or in the body part with the nanoparticles. The temperature increase can also occur in the nanoparticle region and not occur in the body part without the nanoparticles. In this case, the acoustic wave frequency is preferentially lower or larger than $10^{-20}$, $10^{-10}$, $10^{-5}$, $10^{-3}$, $10^{-1}$, 1, 10, $10^3$, $10^5$, $10^{10}$, or $10^{20}$ Hz. In one embodiment of the invention, the acoustic wave frequency is thermal. In this case, the acoustic wave frequency is such that it can induce an increase in temperature or it can induce an increase in temperature of more than $10^3$, $10^2$, 10, 5, 2, 1, or 0.1 °C, wherein the increase in temperature is preferentially occurring (or not) or measured (or not) in the body part without the nanoparticles, or in the nanoparticle region, or in the body part with the nanoparticles. The temperature increase can also occur in the body part without nanoparticles and in the nanoparticle region. In this case, the acoustic wave frequency is preferentially lower or larger than $10^{-20}$, $10^{-10}$, $10^{-5}$, $10^{-3}$, $10^{-1}$, 1, 10, $10^3$, $10^5$, $10^{10}$, or $10^{20}$ Hz.

The invention also relates to nanoparticles for use, wherein the acoustic wave has a penetration depth in the body part, which is larger than 1 cm. In some cases, the acoustic wave can have a penetration depth, which is larger or lower than 0.001, 0.1, 1, 5, 10, 20, 50, or 500 cm, wherein this penetration depth is preferentially the penetration of the acoustic wave through or in the body part, or through or in the nanoparticle region, or through or in 99, 75, 50, 25, 1, 0.1, $10^{-3}$, $10^{-9}$, or $10^{-20}$ % of the body part, or through or in 99, 75, 50, 25, 1, 0.1, $10^{-3}$, $10^{-9}$, or $10^{-20}$ % of the nanoparticle region, or through or in the region separating the equipment generating the acoustic wave and the body part or nanoparticle region.

In one embodiment, the intensity, energy, power, frequency of the acoustic wave decreases, preferentially by a factor of 1.1, 1.2, 1.5, 2, 3, 5, 10, $10^2$, $10^3$, $10^5$, or $10^{10}$, with an increase in the penetration depth, preferentially by a factor of 1.1, 1.2, 1.5, 2, 3, 5, 10, $10^2$, $10^3$, $10^5$, or $10^{10}$.

In one embodiment of the invention, the penetration depth of the acoustic wave is inversely proportional to the acoustic wave frequency. In some cases, the frequency of the acoustic wave can be decreased, preferentially by a factor of 1.1, 1.2, 1.5, 2, 3, 5, 10, $10^2$, $10^3$, $10^5$, or $10^{10}$, preferentially in order to increase the penetration depth of the acoustic wave, preferentially by a factor of 1.1, 1.2, 1.5, 2, 3, 5, 10, $10^2$, $10^3$, $10^5$, or $10^{10}$. Inversely, the frequency of the acoustic wave can be increased, preferentially by a factor of 1.1, 1.2, 1.5, 2, 3, 5, 10, $10^2$, $10^3$, $10^5$, or $10^{10}$, preferentially in order to decrease the penetration depth of the acoustic wave, preferentially by a factor of 1.1, 1.2, 1.5, 2, 3, 5, 10, $10^2$, $10^3$, $10^5$, or $10^{10}$.

In one embodiment of the invention, an acoustic wave volume or acoustic volume is defined as the volume, which is exposed to the acoustic wave or which receives the acoustic wave energy or which undergoes the effects of the acoustic wave.

The invention also relates to nanoparticles for use, wherein the acoustic wave is unfocused. In this case, the acoustic wave can be applied over an acoustic wave volume preferentially comprised in the body part, which is larger than 0.001, 0.01, 0.1, 1, 10, $10^2$, $10^3$, $10^5$, or $10^{10}$ cm$^3$.

In one embodiment of the invention, an unfocused acoustic wave covers more than $10^{-5}$, $10^{-3}$, $10^{-1}$, 1, 10, $10^3$, or $10^5$% of the body part, using less than $10^3$, $10^2$, 10, 5, 2, or 1 application(s) or 1 application spot(s). An application spot can be defined as the acoustic volume that can be covered during a single application of acoustic wave or during one sequence.

The invention also relates to nanoparticles for use, wherein the concentration of the administered nanoparticles is larger than $10^{-3}$ mg per mm$^3$ of body part. In some cases, the nanoparticle concentration is larger than $10^{-9}$, $10^{-7}$, $10^{-5}$, $10^{-3}$, $10^{-1}$, 1, 10, $10^3$, $10^5$, $10^7$, or $10^9$ gram or milligram per mm$^3$ of body part, or $10^9$ gram or milligram per mL of suspension, or $10^9$ gram or milligram per mm$^3$ of matrix or body part (biological or not) comprising the nanoparticles.

The invention also relates to nanoparticles for use, wherein the acoustic wave is focused. In this case, the acoustic wave can be applied over an acoustic wave volume preferentially comprised in the body part, which is smaller than 0.001, 0.1, 1, 10, $10^2$, $10^3$, $10^5$, or $10^{10}$ cm$^3$. In one embodiment of the invention, a focused acoustic wave covers less than $10^{-5}$, $10^{-3}$, $10^{-1}$, 1, 10, $10^3$, or $10^5$% of the body part, using more than $10^3$, $10^2$, 10, 5, 2, or 1 application(s) or 1 application spot(s).

The invention also relates to nanoparticles for use, wherein the nanoparticles induce the destruction of a pathological site of the body part without the destruction of a healthy site surrounding the pathological site. In this case, nanoparticles can induce the destruction of more than 1, 10, $10^3$, $10^6$, $10^9$, or $10^{15}$ pathological cell(s) and/or less than 1, 10, $10^3$, $10^6$, $10^9$, or $10^{15}$ healthy cell(s).

In one embodiment of the invention, the destruction of the pathological site is associated with the death, destruction, denaturation, or inactivation of biological material(s). It can involve (or not): i), immune, pharmaceutical, or metabolic mechanisms, ii), cavitation, iii), heat, or iv), generation of free radicals such as radical oxygen species.

In one embodiment of the invention, cavitation is associated with the production of bubbles. Cavitation can induce a mechanical stress on cells, preferentially cell membranes, and induce cell death. In the presence of the nanoparticles, the size and number of the bubbles can be changed. They can be increased or decreased by a factor larger or smaller than 1.5, 2, 10, 100, $10^3$, or $10^9$. The size and number of the bubbles can tend to be close to that of the nanoparticles exposed to the acoustic wave and differ from the size and number of these nanoparticles by a factor of more or less than $10^9$, $10^5$, $10^3$, $10^2$, or 10.

The invention also relates to nanoparticles for use, wherein the application of acoustic waves on nanoparticles leads to a temperature increase, which is larger in the presence than in the absence of the nanoparticles, or which is larger during $t_1$ than during $t_2$ or $t_3$. The temperature increase occurring during the heating step can be characterized by at least one of the following properties: i), it has a magnitude of more or less than $10^{-5}$, $10^{-3}$, $10^{-1}$, 1, 2, 5, or 10, $10^3$, $10^5$, or $10^9$ °C, preferentially above physiological temperature or above the temperature of the individual before or after the heating step, ii), it leads to an initial temperature increase, also designated as the initial slope of the temperature increase with time, $\Delta T/\delta t$, which is more or less than $10^{-40}$, $10^{-20}$, $10^{-10}$, $10^{-7}$, $10^{-5}$, $10^{-3}$, $10^{-1}$, 1, 2, 5, 10, $10^2$, $10^3$, or $10^5$ °C/sec or $10^5$ °C per sec per gram of nanoparticle or body part or $10^5$ °C per sec per cm$^3$ of nanoparticle or body part, iii), it is such that the maximum temperature reached during a heating step remains below 25, 30, 37, 39, 41, 45, 50, 100, $10^3$, $10^5$, $10^9$, or $10^{11}$ °C, iv), it leads to temperature increase, which is more or less than $10^{-5}$, $10^{-3}$, $10^{-1}$, 1, 2, 5, or 10, $10^3$, $10^5$, or $10^9$ °C above the temperature increase reached by application of the acoustic wave in the absence of the nanoparticles, v), it leads to an initial temperature increase, $\Delta T/\delta t$, which is more or less than $10^{-40}$, $10^{-20}$, $10^{-10}$, $10^{-7}$, $10^{-5}$, $10^{-3}$, $10^{-1}$, 1, 2, 5, 10, $10^2$, $10^3$, or $10^5$ °C/sec or $10^5$ °C per sec per gram of nanoparticle or body part or $10^5$ °C per sec per cm$^3$ of nanoparticle or body part above the initial temperature increase reached by application of the acoustic wave without the nanoparticles.

In another embodiment of the invention, the acoustic wave power, intensity, energy, frequency, or time of application, as well as the nanoparticle concentration, are set at specific values chosen to reach a desired temperature during the heating step, which is preferentially above or below 25, 30, 37, 39, 41, 45, 50, 100, $10^3$, $10^5$, $10^9$, or $10^{11}$ °C. In some cases, to reach the desired temperature, it is possible to increase, preferentially between time $t_1$ and time $t_2$ or $t_3$, the acoustic wave power, intensity, energy, frequency, or time of application, preferentially by a factor of more or less than 1.00001, 1.0001, 1.001, 1.01, 1.1, 1.2, 1.5, 2, 5, 10, $10^3$, $10^5$, $10^{10}$, $10^{20}$, or $10^{40}$. In some cases, to reach the desired temperature, it is also possible to increase the nanoparticle concentration, preferentially between before and after nanoparticle administration, preferentially between inside and outside of the body part, preferentially by a factor of more or less than 1.00001, 1.0001, 1.001, 1.01, 1.1, 1.2, 1.5, 2, 5, 10, $10^3$, $10^5$, $10^{10}$, $10^{20}$, or $10^{40}$.

In one embodiment of the invention, the temperature increase preferentially occurs in the nanoparticle region, or occurs in more than $10^9$, $10^5$, $10^3$, 90, 70, 50, 20, 10, or 1% of the nanoparticle region, and preferentially does not occur outside of this region.

The invention also relates to the non-application of an acoustic wave on nanoparticles or the application of an acoustic wave of low intensity, energy, power, ore frequency on nanoparticles that leads to enhanced cooling. In this case, the non-application of an acoustic wave on nanoparticles or the application of an acoustic wave of low intensity, energy, power, frequency on nanoparticles can lead to a temperature decrease, preferentially occurring during $t_2$ or $t_3$, which is larger in the presence than in the absence of nanoparticles or is larger during $t_2$ or $t_3$ than during $t_1$. The temperature decrease occurring during the cooling step can be characterized by at least one of the following properties: i), it has a magnitude of more or less than $10^{-5}$, $10^{-3}$, $10^{-1}$, 1, 2, 5, 10, $10^3$, $10^5$, or $10^9$ °C, preferentially above physiological temperature or above the temperature of the individual before or after the cooling step, ii), it leads to an initial temperature decrease, also designated as the initial slope of the temperature decrease with time, $\Delta T/\delta t$, which is above or below $10^{-40}$, $10^{-20}$, $10^{-10}$, $10^{-7}$, $10^{-5}$, $10^{-3}$, $10^{-1}$, 1, 2, 5, 10, $10^2$, $10^3$, or $10^5$ °C/sec or $10^5$ °C per sec per gram of nanoparticle or body part or $10^5$ °C per sec per cm$^3$ of nanoparticle or body part, iii), it is such that the minimum temperature reached during a cooling step remains above -273, -150, -100, -75, -50, -30, -10, 0, 5, 10, 25, 30, 37, 39, 41, 45, 50, 100, $10^3$, $10^5$, $10^9$, or $10^{11}$ °C, iv), it leads to a temperature decrease, which is more or less than $10^{-5}$, $10^{-3}$, $10^{-1}$, 1, 2, 5, or 10, $10^3$, $10^5$, or $10^9$ °C above the temperature decrease reached by the non-application of the acoustic wave in the absence of the nanoparticles, v), it leads to an initial temperature decrease, $\Delta T/\delta t$, which is more or less than $10^{-40}$, $10^{-20}$, $10^{-10}$, $10^{-7}$, $10^{-5}$, $10^{-3}$, $10^{-1}$, 1, 2, 5, 10, $10^2$, $10^3$, or $10^5$ °C/sec or $10^5$ °C per sec per gram of nanoparticle or body part or $10^5$ °C per sec per cm$^3$ of nanoparticle or body part, above the initial temperature decrease reached by application of the acoustic wave without the nanoparticles.

In another embodiment of the invention, the acoustic wave power, intensity, energy, frequency, or time of application, as well as the nanoparticle concentration, are set at specific values chosen to reach a desired temperature during the cooling step, which is preferentially above or below -273, -150, -100, -75, -50, -30, -10, 0, 5, 10, 25, 30, 37, 39, 41, 45, 50, 100, $10^3$, $10^5$, $10^9$, or $10^{11}$ °C. In some cases, to reach the desired temperature, it is possible to decrease, preferentially between time $t_1$ and time $t_2$ or $t_3$, the acoustic wave power, intensity, energy, frequency, or time of application, preferentially by a factor of more or less than 1.00001, 1.0001, 1.001, 1.01, 1.1, 1.2, 1.5, 2, 5, 10, $10^3$, $10^5$, $10^{10}$, $10^{20}$, or $10^{40}$. In some cases, to reach the desired temperature, it is also possible to decrease the nanoparticle concentration, preferentially between before and after nanoparticle administration, preferentially between inside and outside of the body part, preferentially by a factor of more or less than 1.00001, 1.0001, 1.001, 1.01, 1.1, 1.2, 1.5, 2, 5, 10, $10^3$, $10^5$, $10^{10}$, $10^{20}$, or $10^{40}$. In one embodiment of the invention, the temperature decrease preferentially occurs in the nanoparticle region, or occurs in more than $10^9$, $10^5$, $10^3$, 90, 70, 50, 20, 10, or 1% of the nanoparticle region, and preferentially does not occur outside of this region.

In one embodiment of the invention, the different parameters that control the desired temperature reached during the heating or cooling step are the frequency, intensity, power, energy, or time of application of the acoustic wave, or the concentration, organization, distribution, size, composition of the nanoparticles. Any of these parameters may be adjusted during the heating or cooling step to reach the desired temperature. Any of these parameters may be fixed or varied during the heating or cooling step.

In one embodiment of the invention, the nanoparticle region is comprised in more or less than $10^{20}$, $10^{10}$, $10^5$, $10^3$, 90, 70, 50, 20, 10, or 1% of the acoustic wave volume. In this case, the temperature increase or heating step can occur in more or less than $10^{20}$, $10^{10}$, $10^5$, $10^3$, 90, 70, 50, 20, 10, or 1% of the acoustic wave volume.

The invention also relates to at least one heating step taking place over a volume, which is less than 50, 10, or 1% of the acoustic wave volume.

The invention also relates to nanoparticles for use, wherein the application of the acoustic wave on nanoparticles induces a temperature increase in the pathological site of more than 1 °C and/or a temperature increase in the healthy site, preferentially surrounding the pathological site, of less than 100 °C.

In one embodiment of the invention, the healthy site surrounds the pathological site when it is located at a distance of less than 1, $10^{-1}$, $10^{-3}$, $10^{-6}$, or $10^{-9}$ m from the pathological site.

In one embodiment of the invention, the temperature increase is preferentially the difference in temperature between the temperatures measured: i), after and before the application of the acoustic wave, ii), during $t_1$ and $t_2$, or iii), during $t_1$ and $t_3$. It can be equal to, larger than or lower than 0.001, 0.01, 0.1, 1, 10, $10^2$, $10^3$, or $10^5$ °C. The temperature increase can also be more than 0.001, 0.01, 0.1, 1, 10, $10^2$, $10^3$, or $10^5$ °C more important in the presence than in the absence of the nanoparticles. It can also be less than 0.001, 0.01, 0.1, 1, 10, $10^2$, $10^3$, or $10^5$ °C more important in the presence than in the absence of the nanoparticles.

The invention also relates to nanoparticles for use, wherein the application of the acoustic wave on nanoparticles induces a series of temperature increases of the body part followed by temperature decreases of the body part. Temperature increase preferentially occurs during $t_1$, while temperature decrease preferentially takes place during $t_2$ or $t_3$. The number of temperature increase or temperature decrease is preferentially larger than 1, 5, 10, $10^2$, or $10^3$. In one embodiment of the invention, several or more than 1, 5, 10, or 100 sequences, or a series of temperature increases followed by temperature decreases is a treatment session or session. A treatment session can last more or less than 1, 5, 10, 20, 50, $10^2$, $10^3$, or $10^5$ minute(s). A medical treatment can comprise more than 1, 2, 5, 10, or $10^3$ treatment session(s), where each treatment session can be separated by a lapse of time of more or less than 1, 5, 10, $10^2$, $10^3$, $10^5$, $10^{10}$, or $10^{50}$ minute(s). The lapse of time separating two sessions is preferentially longer than the times $t_1$, $t_2$, $t_3$, $t_1+t_2$, or $t_1+t_3$, preferentially by a factor of more or less than 1.001, 1.01, 1.1, 1.2, 1.5, 2, 5, 10, 20, 50, 100, $10^3$, $10^5$, or $10^9$.

In one embodiment of the invention, the duration of a session or the time separating two sessions is such, preferentially sufficiently long, that a mechanism of treatment can occur.

A mechanism of treatment such as that of tumor destruction can involve the immune system, an apoptotic mechanism, or a by stander effect. It preferentially enables to destroy pathological cells at a distance from the nanoparticle or nanoparticle region, which is more or less than 5, 2, 1, $10^{-1}$, $10^{-3}$, $10^{-3}$, $10^{-6}$, or $10^{-9}$ m. In some cases, such mechanism can be reactivated during each session or sequence by applying or re-applying the acoustic wave and/or deactivated by stopping or re-stopping the application of the acoustic wave.

The invention also relates to nanoparticles for use, wherein the application of acoustic waves on the body part leads to a temperature increase of the body part, which is at least 1% larger in the presence than in the absence of the nanoparticles. In some cases, the temperature increase can be more or less than 500, 200, 100, 75, 50, 10, or 1% larger in the presence than in the absence of the nanoparticles.

The invention also relates to nanoparticles for use, wherein the temperature increase of the body part occurs within less than 50% of the body part. In some cases, the temperature increase can occur within more or less than $10^{20}$, $10^{10}$, $10^5$, $10^3$, 500, 200, 90, 70, 50, 10, or 1% of the body part.

The invention also relates to nanoparticles for use, wherein the temperature increase of the body part occurs within more than $10^{20}$, $10^{10}$, $10^5$, $10^3$, 500, 200, 90, 70, 50, 10, or 1% of the volume occupied by the nanoparticles in the body part.

In one embodiment of the invention, the application of the acoustic wave on nanoparticles leads to an initial temperature increase $(\Delta T/\delta t)_{(N)}$ with at least one of the following properties: i), $(\Delta T/\delta t)_{(N)}$ is larger or lower than $10^{-7}$, $10^{-5}$, $10^{-3}$, $10^{-1}$, 1, 10, $10^3$, or $10^5$ °C/sec or $10^5$ °C per sec per gram of nanoparticle or body part or $10^5$ °C per sec per cm$^3$ of nanoparticle or body part, ii), $(\Delta T/\delta t)_{(N)}$ is more than a factor of 1.2, 1.5, 2, 5, 10, $10^3$, $10^5$, or $10^9$ more important in than $(\Delta T/\delta t)_{(WN)}$, measured in the absence of the nanoparticles, iii), $(\Delta T/\delta t)_{(N)}$ is lower by a factor of 1.2, 1.5, 2, 5, 10, $10^3$, $10^5$, or $10^9$ than $(\Delta T/\delta t)_{(WN)}$. $(\Delta T/\delta t)_{(N)}$ and $(\Delta T/\delta t)_{(WN)}$ can be the temperature increase measured during the initial application of the acoustic wave energy, *i.e.* during less than $10^3$, $10^2$, 10, 1, $10^{-2}$, $10^{-3}$, $10^{-6}$, or $10^{-9}$ seconds, preferentially following the beginning of the application of the acoustic wave energy. $(\Delta T/\delta t)_{(N)}$ can represent a temperature variation by more than 1, 5, 10, 50, 75, $10^2$, or $10^3$ %, during a time of application of the acoustic wave designated as $t_i$.

In one embodiment of the invention, the application of the acoustic wave energy on nanoparticles leads to a saturating temperature (ST) with at least one of the following properties: i), ST is larger or lower than $10^{-7}$, $10^{-5}$, $10^{-3}$, $10^{-1}$, 1, 10, $10^3$, or $10^5$ °C, ii), ST is more than a factor of 1.2, 1.5, 2, 5, 10, $10^3$, $10^5$, or $10^9$ more important in the presence than in the absence of the nanoparticles, iii), ST is less than a factor of 1.2, 1.5, 2, 5, 10, $10^3$, $10^5$, or $10^9$ more important in the presence than in the absence of the nanoparticles. The saturating temperature can be the maximum temperature that can be reached during the time of application of the acoustic wave or represent a temperature that does not vary by more than 1, 5, 10, 50, 75, $10^2$, or $10^3$ %, during a time of application of the acoustic wave designated as $t_S$. In some cases, the saturating temperature can be the desired temperature.

The time $t_S$ preferentially follows the time $t_i$.

In one embodiment of the invention, the application of the acoustic wave is stopped as soon as the saturating temperature is reached, preferentially to avoid overheating.

In one embodiment of the invention, the ratio $t_S/t_i$ is larger or smaller than $10^{-9}$, $10^{-6}$, $10^{-3}$, $10^{-1}$, 1, 10, $10^3$, $10^6$, or $10^9$.

In another embodiment of the invention, the heating step lasts $t_i+t_s$, where $t_s$ is preferentially smaller than $t_i$, where $t_i/(t_i+t_s)$ is preferentially larger than $10^{-9}$, $10^{-7}$, $10^{-5}$, $10^{-4}$, $10^{-3}$, $10^{-2}$, $10^{-1}$, 0.5, 0.75, or 0.9.

**EXAMPLE 1:**

MATERIALS AND METHODS:

Nanoparticles: We used as nanoparticles: i), magnetosomes extracted from magnetotactic bacteria composed of maghemite that were coated with carboxy-methyl-dextran using an adapted and improved protocol described in patent PCT/FR2016/000095 incorporated in reference (example 8), designated as Magnetosome(s) ii), nanoparticles composed of iron oxide of sizes 35 $\pm$ 13 nm purchased from Sigma designated as Sigma nanoparticles (Ref: 637106-25G, Lot #MKBK2270V), iii), superparamagnetic nanoparticles composed of iron oxide of 20 nm purchased from Micromod designated as SPION20 (nanomag®-D-spio 20, Ref: 79-02-201), iv), superparamagnetic nanoparticles composed of iron oxide of 50 nm purchased from Micromod designated as SPION50 (synomag□-D 50, Ref: 104-000-501), v), superparamagnetic nanoparticles composed of iron oxide of 100 nm purchased from Micromod designated as SPION100 (nanomag®-D-spio 100, Ref: 79-00-102).

Preparation of samples containing nanoparticles inserted in tissue or dispersed in water: For heating experiments in tissues, 10 μl of suspensions containing water alone or 204 μg in iron of nanoparticles (Magnetosome(s) and Sigma or Sigma nanoparticle(s)) were inserted homogenously in 4.5 cm$^3$ of liver tissue leading to a concentration of 45 μg in iron of nanoparticles per cm$^3$ of liver tissue. For heating experiments in aqueous conditions, 100 μl of water alone or 100 μl of water mixed with 100 μg in iron of nanoparticles (Magnetosome, Sigma, SPION20, SPION50, SPION100) were dispersed in a 200 μl Eppendorf.

Heating apparatus generating ultrasound: Samples made of tissues with/without nanoparticles or water with/without nanoparticles were exposed to ultrasound of energy 0.5, 1, or 1.5 W/cm$^3$, and frequency 3 MHz, during 10 minutes. To apply the ultrasound, we used a phyaction 190i ultrasound generator with a transducer of surface 4 cm$^2$. The ultrasound power indicated in the example corresponds to that read on the 190i ultrasound generator and not to an ultrasound power measured with an external probe. We used an ultrasound gel (Winelec, Ref: 1741/WINELEC) located between the transducer and the samples to favor the transmission of the ultrasounds.

Measurement of temperature: We used an infrared camera (EasIRTM-2, Optophase) positioned 13 cm above the transducer to measure the spatial distribution in temperature as a function of time during the experiments. We measured the temperature distribution at the following time points: 0 sec., 30 sec., 1 min., 2 min., 3 min., 4 min., 5 min., 6 min., 7 min., 8 min., 9 min., and 10 min. We only considered the maximum temperature recorded at each time point.

RESULTS AND DISCUSSION:

a) Non sequential heating experiment in tissues:

Figure 1 shows ΔT, the difference in temperature between the tissue with the nanoparticles and the tissue without the nanoparticles, measured at each time point, and for an ultrasound power of 0.5 W/cm$^2$ (Figure 1(a)), 1 W/cm$^2$ (Figure 1(b)), 1.5 W/cm$^2$ (Figure 1(c)). At the three different tested powers, ΔT is positive indicating that the temperature increase is more important for the tissue containing nanoparticles than for tissue without the nanoparticles. At the lowest power of 0.5 W/ cm$^2$, Sigma nanoparticles produce more heat than Magnetosomes, while at 1.5 W/cm$^2$, the opposite behavior is observed with Magnetosomes producing more heat than Sigma nanoparticles.

For the magnetosomes mixed in tissue and exposed to different ultrasound powers of 0.5, 1, or 1.5 W/cm$^2$, we have also estimated the values of $\Delta T_{10minreal(M)}$, which is equal to $\Delta T_{10min(M)}$ - $\Delta T_{10min(W)}$, where $\Delta T_{10min(M)}$ and $\Delta T_{10min(W)}$ are the temperature increases observed after 10 minutes of ultrasound application for the samples containing tissue with the magnetosomes and tissue without the magnetosomes, respectively. We observed that $\Delta T_{10minreal(M)}$ increases from 6 °C at 0.5 W/cm$^2$ to 28 °C at 1.5 W/cm$^2$ (table 1). We also estimated the percentage in temperature rise, Temperature rise $_{(M)}$, expressed using the formula Temperature rise$_{(M)}$=$(\Delta T_{10min(M)}/\Delta T_{10min(W)}$-1).100. It increases from 37% at 0.5 W/cm$^2$ to 100% at 1.5 W/cm$^2$ (table 1). We also estimated the value of the specific absorption rate of the magnetosomes inserted in tissue, SAR$_{real(M)}$, expressed in watt per gram of magnetosomes in iron (W/g$_{Fe}$), using the formula SAR$_{real(M)}$=Slope$_{real(M)}$.C$_v$/C$_{nano}$, where Slope$_{real(M)}$=Slope$_{(M)}$-Slope$_{(W)}$, with Slope$_{(M)}$ and Slope$_{(W)}$ representing the initial slopes of the temperature variations with time deduced from the plots of Figures 1(a) to 1(c), C$_v$ = 4.2 J.K$^{-1}$g$^{-1}$ is the specific heat of water and C$_{nano}$ is the nanoparticle concentration in gram of nanoparticles per mL of water. SAR$_{real(M)}$ increases from 5-12 W/g$_{Fe}$ at 0.5-1 W/cm$^2$ to 71 W/g$_{Fe}$ at 1.5 W/cm$^2$ (table 1). We also estimated the percentage in slope rise, Slope rise $_{(M)}$, expressed using the formula Slope rise $_{(M)}$ = [(Slope$_{(M)}$/Slope$_{(W)}$)-1].100. It increases from 9-47% at 0.5-1 W/cm$^2$ to 124% at 1.5 W/cm$^2$.

For Sigma nanoparticles mixed in tissue and exposed to different ultrasound powers of 0.5, 1, or 1.5 W/cm$^2$, we have also estimated the values of $\Delta T_{10minreal(S)}$, which is equal to $\Delta T_{10min(S)}$-$\Delta T_{10min(W)}$, where $\Delta T_{10min(S)}$ and $\Delta T_{10min(W)}$ are the temperature increases observed after 10 minutes of ultrasound application for the samples containing tissue with the Sigma nanoparticles and tissue without the Sigma nanoparticles, respectively. We observed that

$\Delta T_{10minreal(S)}$ decreases from 14 °C at 0.5 W/cm$^2$ to 6-7 °C at 1-1.5 W/cm$^2$ (table 1). We also estimated the percentage in temperature rise, Temperature rise $_{(S)}$, expressed using the formula Temperature rise $_{(S)}$ = ($\Delta T_{10min(S)}/\Delta T_{10min(W)}$-1).100. It decreases from 90% at 0.5 W/cm$^2$ to 17-26% at 1-1.5 W/cm$^2$ (table 1). We also estimated the value of the specific absorption rate of the Sigma nanoparticles inserted in tissue, SAR$_{real(S)}$, expressed in watt per gram of Sigma nanoparticles in iron (W/g$_{Fe}$), using the formula SAR$_{real(S)}$ = Slope$_{real(S)}$·C$_v$/C$_{nano}$, where Slope$_{real(S)}$ represent the initial slopes of the temperature variations with time deduced from the plots of Figures 1(a) to 1(c) for sigma nanoparticles, C$_v$ = 4.2 J.K$^{-1}$g$^{-1}$ is the specific heat of water and C$_{nano}$ is the nanoparticle concentration in gram of Sigma nanoparticles per mL of water. SAR$_{real(S)}$ remains at 16-28 W/g$_{Fe}$ between 0.5 and 1.5 W/cm$^2$ (table 1). We also estimated the percentage in slope rise, Slope rise $_{(S)}$, expressed using the formula Slope rise $_{(S)}$=(Slope$_{(S)}$/Slope$_{(W)}$-1).100. It decreases from 118% at 0.5 W/cm$^2$ to 30-36% at 1-1.5 W/cm$^2$.

b) <u>Non-sequential heating experiments in water:</u>

Figures 2(a), 2(b), 2(c), 2(d), and 2(e), show $\Delta T$, the difference between the temperature of the suspension containing the different nanoparticles dispersed in water and temperature of water without the nanoparticles, when the different suspensions are exposed to ultrasounds of 0.5, 1, or 1.5 W/cm$^2$ during 10 minutes. Figures 2(a), 2(b), 2(c), 2(d), and 2(e) show $\Delta T$ as a function of time for Magnetosomes, Sigma, SPION50, SPION100, and SPION20, respectively. For the different nanoparticles and the three different tested powers, $\Delta T$ is positive indicating that the temperature increase is more important for nanoparticles dispersed in water than for water alone.

For the magnetosomes mixed in water and exposed to different ultrasound powers of 0.5, 1, or 1.5 W/cm$^2$, we have estimated the values of $\Delta T_{10minreal(M)}$, which is equal to $\Delta T_{10min(M)}$-$\Delta T_{10min(W)}$, where $\Delta T_{10min(M)}$ and $\Delta T_{10min(W)}$ are the temperature increases observed after 10 minutes of ultrasound application for the samples containing tissue with the magnetosomes and tissue without the magnetosomes, respectively. We observed that $\Delta T_{10minreal(M)}$ remains at 3 to 9 °C between 0.5 W/cm$^2$ and 1.5 W/cm$^2$ (table 2), smaller values of $\Delta T_{10minreal(M)}$ than those observed in tissue at 1.5 W/cm$^2$ (table 1). We also estimated the percentage in temperature rise, Temperature rise $_{(M)}$, expressed using the formula Temperature rise $_{(M)}$ = ($\Delta T_{10min(M)}/\Delta T_{10min(W)}$-1).100. It decreases from 37-43% at 0.5-1 W/cm$^2$ down to 10% at 1.5 W/cm$^2$ (table 2) and is also smaller than Temperature rise $_{(M)}$ measured in tissue at 1.5 W/cm$^2$ (table 1). We also estimated the value of the specific absorption rate of the magnetosomes dispersed in water, SAR$_{real(M)}$, expressed in watt per gram of magnetosomes in iron (W/g$_{Fe}$), using the formula SAR$_{real(M)}$ = Slope$_{real(M)}$·C$_v$/C$_{nano}$, where Slope$_{real(M)}$ represents the initial slopes of the temperature variations with time deduced from the plots of Figure 2(a), C$_v$ = 4.2 J.K$^{-1}$g$^{-1}$ is the specific heat of water and C$_{nano}$ is the Magnetosome concentration in gram of magnetosomes per mL of water. SAR$_{real(M)}$ increases from 294 W/g$_{Fe}$ at 0.5 W/cm$^2$ to 424 W/g$_{Fe}$ at 1.5 W/cm$^2$ (table 2), higher values than those measured in tissue (table 1). We also estimated the percentage in slope rise, Slope rise $_{(M)}$, expressed using the formula Slope rise $_{(M)}$ = (Slope$_{(M)}$/Slope$_{(W)}$-1).100. It remains at 16-24% at 0.5-1.5 W/cm$^2$ (table 2), smaller values than 124% deduced in tissue at 1.5 W/cm$^2$ (table 1).

For Sigma nanoparticles dispersed in water and exposed to different ultrasound powers of 0.5, 1, or 1.5 W/cm$^2$, we have also estimated the values of $\Delta T_{10minreal(S)}$, which is equal to $\Delta T_{10min(S)}$-$\Delta T_{10min(W)}$, where $\Delta T_{10min(S)}$ and $\Delta T_{10min(W)}$ are the temperature increases observed after 10 minutes of ultrasound application for the samples containing Sigma nanoparticles dispersed in water and water without the Sigma nanoparticles, respectively. We observed that $\Delta T_{10minreal(S)}$ remains at 6-12 °C for ultrasound energies of 0.5-1.5 W/cm$^2$ (table 2). We also estimated the percentage in temperature rise, Temperature rise $_{(S)}$, expressed using the formula Temperature rise $_{(S)}$=($\Delta T_{10min(S)}/\Delta T_{10mm(W)}$-1).100. It remains at 31-60% for powers of 0.5-1.5 W/cm$^2$ (table 2). We also estimated the value of the specific absorption rate of the Sigma nanoparticles inserted in tissue, SAR$_{real(S)}$, expressed in watt per gram of Sigma nanoparticles in iron (W/g$_{Fe}$), using the formula SAR$_{real(S)}$ = Slope$_{real(S)}$·C$_v$/C$_{nano}$, where Slope$_{real(S)}$ represents the initial slopes of the temperature variations with time deduced from the plots of Figure 2(b) for Sigma nanoparticles, C$_v$ = 4.2 J.K$^{-1}$g$^{-1}$ is the specific heat capacity of water and C$_{nano}$ is the nanoparticle concentration in gram of Sigma nanoparticles in iron per mL of water. SAR$_{real(S)}$ increases from 0 W/g$_{Fe}$ at 0.5 W/cm$^2$ to 2686 W/g$_{Fe}$ at 1.5 W/cm$^2$ (table 2). We also estimated the percentage in slope rise, Slope rise $_{(S)}$, expressed using the formula Slope rise $_{(S)}$ = (Slope$_{(S)}$/Slope$_{(W)}$-1).100. It increases from 0-8% at 0.5-1 W/cm$^2$ to 99% at 1.5 W/cm$^2$.

For SPION50, SPION100, and SPION20 nanoparticles dispersed in water and exposed to different ultrasound powers of 0.5, 1, or 1.5 W/cm$^2$, we have also estimated the values of $\Delta T_{10minreal(S50)}$, $\Delta T_{10minreal(S100)}$, and $\Delta T_{10minreal(S20)}$, which are equal to $\Delta T_{10min(S50)}$-$\Delta T_{10min(W)}$, $\Delta T_{10min(S100)}$-$\Delta T_{10min(W)}$ and $\Delta T_{10min(S20)}$ - $\Delta T_{10min(W)}$, respectively. $\Delta T_{10min(S50)}$, $\Delta T_{10min(S100)}$, $\Delta T_{10min(S20)}$ and $\Delta T_{10min(W)}$ are the temperature increases observed after 10 minutes of ultrasound application for the samples containing SPION50, SPION100, and SPION20 nanoparticles dispersed in water and water without nanoparticles, respectively. We observed that $\Delta T_{10minreal(S50)}$, $\Delta T_{10minreal(S100)}$, and $\Delta T_{10minreal(S20)}$, remain at 0-7 °C for ultrasound energies of 0.5-1.5 W/cm$^2$ (table 2). We also estimated the percentage in temperature rise, Temperature rise $_{(S)}$, expressed using the formula Temperature rise $_{(S50)}$ = ($\Delta T_{10min(S50)}/\Delta T_{10min(W)}$-1).100 for SPION50, Temperature rise $_{(S100)}$ = ($\Delta T_{10min(S100)}/\Delta T_{10min(W)}$-1).100 for SPION100, Temperature rise $_{(S20)}$ =

$(\Delta T_{10min(S20)}/\Delta T_{10min(W)}$-1).100 for SPION20. It remains at 1-35% for powers of 0.5-1.5 W/cm$^2$ for the different SPION (table 2). We also estimated the value of the specific absorption rate of the SPION50, SPION100, and SPION20 nanoparticles inserted in tissue, SAR$_{real(S50)}$, SAR$_{real(S100)}$, SAR$_{real(S20)}$, expressed in watt per gram of SPION50, SPION100, and SPION20 nanoparticles in iron (W/g$_{Fe}$). For that, we used the formula SAR$_{real(S50)}$ = Slope$_{real(S50)}$.C$_v$/C$_{nano}$, SAR$_{real(S100)}$ = Slope$_{real(S100)}$.C$_v$/C$_{nano}$, SAR$_{real(S20)}$ = Slope$_{real(S20)}$.C$_v$/C$_{nano}$ for SPION50, SPION100, and SPION20 nanoparticles, respectively. Slope$_{real(S50)}$, Slope$_{real(S100)}$, Slope$_{real(S20)}$ represent the initial slopes of the temperature variations with time deduced from the plots of Figure 2(c) for SPION50, of Figure 2(d) for SPION100, and of Figure 2(e) for SPION20, where C$_v$ = 4.2 J.K$^{-1}$g$^{-1}$ is the specific heat of water and C$_{nano}$ is the nanoparticle concentration in gram of SPION50, SPION20, or SPION100 nanoparticles in iron per mL of water. SAR$_{real(S20)}$, SAR$_{real(S50)}$, and SAR$_{real(S100)}$ increase from 0-677 W/g$_{Fe}$ at 0.5-1 W/cm$^2$ to 787-2795 W/g$_{Fe}$ at 1.5 W/cm$^2$ (table 2). We also estimated the percentage in slope rise, Slope rise $_{(S20)}$, Slope rise $_{(S50)}$, Slope rise $_{(S100)}$, expressed using the formula Slope rise $_{(S20)}$=(Slope$_{(S20)}$/Slope$_{(W)}$-1).100 for SPION20, Slope rise $_{(S50)}$=(Slope$_{(S50)}$/Slope$_{(W)}$-1).100 for SPION50 and Slope rise (S$_{100}$)=(Slope$_{(S100)}$/Slope$_{(W)}$-1).100 for SPION100. It remains at 0-104% between 0.5 and 1.5 W/cm$^2$.

c) <u>Sequential heating experiments in water:</u>

Eppendorf containing 500 µg of Magnetosomes dispersed in 100 µl of water were exposed sequentially to ultrasounds. Figure 3(a) shows 13 sequences (SQ1 to SQ13) consisting for each of them in the application of an ultrasound of power 1.5 W/cm$^2$ and frequency 3 MHz during time t$_1$ followed by the non-application of an ultrasound during times t$_2$. The time t$_1$ corresponds to the time necessary to reach a desired targeted temperature of 43.5 $\pm$ 1.5 °C during the heating step, while the time t$_2$ corresponds to the time necessary to cool down the sample from 43.5 $\pm$ 1.5 °C to 34.5 $\pm$ 0.5 °C during the cooling step. The values of t$_1$ and t$_2$ are given in table 3 for the different sequences. The average frequency of the sequences, 1/(t$_{1av}$+t$_{2av}$), where t$_{1av}$ and t$_{2av}$ represent the average values of t$_1$ and t$_2$ over the 13 sequences, was estimated as 33 mHz. Figure 3(b) shows the variation of $\Delta T$, the difference in temperature between the temperature of the tube containing water with the Magnetosome and the temperature of the tube containing water without the Magnetosome, as a function of time. $\Delta T$ is positive indicating that the temperature increase is more important in the tube containing water with magnetosomes than in the tube containing water alone without magnetosomes during all 13 sequences. Furthermore, we are able to repeat the heating and cooling steps due to the presence of Magnetosome a large number of times (13) as seen in Figure 3(b), suggesting that the ultrasound are not damaging the Magnetosomes or are not strongly undermining the heating power of the magnetosomes. The heating steps are more important in magnitude during the two first sequences, which may be attributed to better magnetosome dispersion and lower magnetosome aggregation during the first two sequences than during the other remaining sequences. We also observe that the sequences can be repeated with heating and cooling times that do not vary by more than 53 % between the different sequences (table 3).

<u>CONCLUSION:</u>

We can draw the following conclusion from this example:

(i), The values of $\Delta T$, the difference in temperature between the temperature of nanoparticles in tissues or water exposed to ultrasounds and the temperature of tissue or water alone exposed to ultrasounds, is always positive, indicating that the different nanoparticles (Magnetosome, Sigma, SPION20, SPION50, SPION100) enhance the heating efficacy of ultrasound in the tested conditions (ultrasound frequency = 3 MHz, ultrasound power = 0.5-1.5 W/cm$^2$, nanoparticle concentration varied between 60µg/mL and 8 mg/mL and nanoparticles either inserted in tissue or dispersed in water).
(ii), Nanoparticle SAR values due to the application of the ultrasounds in the tissue are the highest for the magnetosomes.
(iii), Using Magnetosomes, we can produce sequences consisting in heating steps (application of ultrasounds on Magnetosomes) followed by cooling steps (non-application of ultrasounds on Magnetosomes), with enhanced magnitudes of heating and cooling compared with heating and cooling steps obtained without the Magnetosomes (Figures 3(a) and 3(b)).

**EXAMPLE 2:**

Figure 4 shows that when 100 µl of a suspension of BNF-Starch nanoparticles mixed in water are exposed to an alternating magnetic field of average strength 30 mT and frequency 196 kHz during 30 minutes, the SAR increases from 4 Watt per gram of iron comprised in nanoparticles for a concentration of 500 µg of iron comprised in nanoparticles per mL up to 114 Watt per gram of iron comprised in nanoparticles for a concentration of 5 mg of iron comprised in nanoparticles per mL. The SAR increases by factor of 29 for an increase in nanoparticle concentration by a factor of 10. Above 5 mg/mL, the SAR saturates at 110 Watt per gram of iron comprised in nanoparticles.

**Table 1**. For 210 $\mu$g in iron of nanoparticles Magnetosome inserted in 4.6 cm$^3$ of tissue exposed to ultrasounds of frequency 3 MHz and power 0.5 W/cm$^2$, 1 W/cm$^2$, 1.5 W/cm$^2$, (Slope$_{(M)}$) designs the slope at the origin of the temperature variation with time of magnetosomes mixed with tissue (Slope$_{real(M)}$) designing the difference between slope at the origin of the temperature variation with time of magnetomes mixed with tissue (Slope$_{(M)}$) and the slope at the origin of the temperature variation with time of the tissue without the nanoparticles (Slope$_{(w)}$). Slope$_{realN(M)}$ is Slope$_{real(M)}$ divided by the magnetosome concentration in gram of iron comprised in magnetosomes per mL. Slope rise (Slope rise (M)) designates the percentage in slope rise estimated using the formula for magnetosomes: Slope rise $_{(M)}$ (%) = ((Slope$_{(M)}$/Slope$_{(W)}$)-1)*100. The specific absorption rate of magnetosomes (SAR$_{(M)}$), estimated in watt per gram of magnetosomes is deduced from the values of Slope $_{(M)}$, using the formula: SAR$_{(M)}$ = C$_v$.Slope$_{(M)}$/C$_{nano}$, where C$_v$ = 4.2 J.K$^{-1}$.g$^{-1}$ is the specific heat of water and C$_{nano}$ is the magnetosome concentration in gram of magnetosomes per cm$^3$ of tissue. The variation in temperature between the initial temperature measured before the application of the ultrasound and the temperature measured after 10 minutes of application of the ultrasound is designated as $\Delta T_{10min(M)}$ for magnetosomes. The difference between $\Delta T_{10min(M)}$ and $\Delta T_{10min(w)}$ is designated as $\Delta T_{10min,real(M)}$. The percentage in temperature rise is estimated for Magnetosomes using the formula: Temperature rise $_{(M)}$ (%) = (($\Delta T_{10min(M)}$/$\Delta T_{10min(W)}$)-1)*100 for Magnetosomes. For 210 $\mu$g in iron of Sigma nanoparticles inserted in 4.6 cm$^3$ of tissue exposed to ultrasounds of frequency 3 MHz and power 0.5 W/cm$^2$, 1 W/cm$^2$, 1.5 W/cm$^2$, (Slope$_{(S)}$) designs the slope at the origin of the temperature variation with time of Sigma nanoparticles mixed with tissue (Slope$_{real(S)}$) designs the difference between slope at the origin of the temperature variation with time of Sigma nanoparticles mixed with tissue (Slope$_{(S)}$) and the slope at the origin of the temperature variation with time of the tissue without the nanoparticles (Slope$_{(w)}$). Slope$_{realN(S)}$ is Slope$_{real(S)}$ divided by the Sigma nanoparticle concentration in gram of iron comprised in Sigma nanoparticles per mL. Slope rise (Slope rise (S)) designates the percentage in slope rise estimated using the formula for magnetosomes: Slope rise $_{(S)}$ (%) = ((Slope$_{(S)}$/Slope$_{(W)}$)-1)*100. The specific absorption rate of Sigma nanoparticles (SAR$_{(S)}$), estimated in watt per gram of Sigma nanoparticles is deduced from the values of Slope $_{(S)}$, using the formula: SAR$_{(S)}$ = C$_v$.Slope$_{(S)}$/C$_{nano}$, where C$_v$ = 4.2 J.K$^{-1}$.g$^{-1}$ is the specific heat of water and C$_{nano}$ is the Sigma nanoparticle concentration in gram of Sigma nanoparticles per cm$^3$ of tissue. The variation in temperature between the initial temperature measured before the application of the ultrasound and the temperature measured after 10 minutes of application of the ultrasound is designated as $\Delta T_{10min(S)}$ for Sigma nanoparticles. The difference between $\Delta T_{10min(S)}$ and $\Delta T_{10min(w)}$ is designated as $\Delta T_{10min,reals(S)}$. The percentage in temperature rise is estimated for Sigma nanoparticles using the formula:

$$\text{Temperature rise } _{(S)} (\%) = ((\Delta T_{10min(S)}/\Delta T_{10min(W)})-1)*100 \text{ for Sigma nanoparticles.}$$

**Table 1**

| Heating on tissue | | | | | | | | | | | | |
|---|---|---|---|---|---|---|---|---|---|---|---|---|
| **Water** | | | | **Magnetosome** | | | | **Sigma** | | | | |
| | 0.5 W/cm² | 1 W/cm² | 1.5 W/cm² | | 0.5 W/cm² | W/cm² | 1.5 W/cm² | | 0.5 W/cm² | 1 W/cm² | 1.5 W/cm² | |
| Slope $_{(w)}$ (°C/sec) | 0.063 | 0.145 | 0.142 | Slope $_{(M)}$ (°C/sec) | 0.092 | 0.158 | 0.318 | Slope $_{(S)}$ (°C/sec) | 0.137 | 0.197 | 0.184 | |
| | | | | Slope$_{real(M)}$ (°C/sec) | 0.029 | 0.013 | 0.177 | Slope$_{real (S)}$ (°C/sec) | 0.074 | 0.052 | 0.042 | |
| | | | | Stope$_{real\ N(M)}$ (mL.°C/sec/g$_{Fe}$) | 3 | 1 | 17 | Slope$_{real\ N(S)}$ (°C/sec/g$_{Fe}$) | 7 | 5 | 4 | |
| | | | | Slope rise $_{(M)}$ (%) | 47 | 9 | 124 | Slope rise $_{(S)}$ (%) | 118 | 36 | 30 | |
| | | | | SAR $_{(M)}$ (W/g$_{Fe}$) | 37 | 64 | 128 | SAR $_{(S)}$ (W/g$_{Fe}$) | 52 | 75 | 70 | |
| | | | | SAR$_{real\ (M)}$ (W/g$_{Fe}$) | 12 | 5 | 71 | SAR$_{real\ (S)}$ (W/G$_{Fe}$) | 28 | 20 | 16 | |
| $\Delta T_{10\ min\ (w)}$ (°C) | 16 | 32 | 28 | $\Delta T_{10min\ (M)}$ (°C) | 22 | 40 | 56 | $\Delta T_{10min\ (S)}$ (°C) | 30 | 38 | 35 | |
| | | | | $\Delta T_{10\ min\ real\ (M)}$ (°C) | 6 | 8 | 28 | $\Delta T_{10min\ real\ (S)}$ (°C) | 14 | 6 | 7 | |
| | | | | Temperature rise $_{(M)}$ (%) | 37 | 25 | 100 | Temperature rise $_{(S)}$ (%) | 90 | 17 | 26 | |

**Table 2**. For 100 μg in iron of nanoparticles (Magnetosome, Sigma, SPION20, SPION50, SPION100) dispersed in 100 μl of water exposed to ultrasounds of frequency 3 MHz and power 0.5 W/cm$^2$, 1 W/cm$^2$, 1.5 W/cm$^2$, slopes at the origin of the temperature variation with time of the different nanoparticles dispersed in water, designated as Slope$_{(M)}$ for Magnetosome, Slope$_{(S)}$ for Sigma nanoparticles, Slope$_{(S20)}$ for SPION20, Slope$_{(S50)}$ for SPION50, Slope$_{(S100)}$ for SPION100. The difference between the slope at the origin of the temperature variation with time of nanoparticles dispersed in water and the slope at the origin of the temperature variation with time of water without the nanoparticles is designated by Slope$_{real(M)}$ for Magnetosome, Slope$_{real(S)}$ for Sigma nanoparticles, Slope$_{real(S20)}$ for SPION20, Slope$_{real(S50)}$ for SPION50, Slope$_{real(S100)}$ for SPION100. Values of slope rise in percentage estimated using the formula: Sloperise$_{(M)}$=((Slope$_{(M)}$/Slope$_{(W)}$-1)*100 for magnetosomes; Sloperise$_{(S20)}$=((Slope$_{(S20)}$/Slope$_{(W)}$)-1)*100 for SPION20; Sloperise$_{(S50)}$=((Slope$_{(S50)}$/Slope$_{(W)}$-1)*100 for SPION50; Sloperise$_{(S100)}$=((Slope$_{(S100)}$/Slope$_{(W)}$)-1)*100 for SPION100. The specific absorption rate (SAR), measured in watt per gram of nanoparticles, deduced from the values of Slope, using the formula: SAR$_{(M)}$ = C$_v$.Slope$_{(M)}$/C$_{nano}$ for Magnetosome, SAR(S) = C$_v$.Slope$_{(S)}$/C$_{nano}$ for Sigma nanoparticles, SAR$_{(S20)}$ = C$_v$.Slope$_{(S20)}$/C$_{nano}$ for SPION20, SAR$_{(S50)}$ = C$_v$.Slope$_{(S50)}$/C$_{nano}$ for SPION50, and SAR$_{(S100)}$ = C$_v$.Slope$_{(S100)}$/C$_{nano}$ for SPION100, where C$_v$ = 4.2 J.K$^{-1}$g$^{-1}$ is the specific heat of water, C$_{nano}$ is the nanoparticle (Magnetosome, Sigma, SPION20, SPION50, or SPION100) concentration in gram of nanoparticles per mL of water. Slope$_{(M)}$, Slope(s), Slope$_{(S20)}$, Slope$_{(S50)}$, Slope$_{(S100)}$ are the initial slopes of the temperature variation for Magnetosome, Sigma nanoparticles, SPION20, SPION50, and SPION100. The real specific absorption rate (SAR$_{real}$), measured in watt per gram of nanoparticles is deduced from the values of Slope$_{real}$, using the formula: SAR$_{real(M)}$=C$_v$.Slope$_{real(M)}$/C$_{nano}$ for magnetosomes, SAR$_{real(S)}$=C$_v$.Slope$_{real(S)}$/C$_{nano}$ for Sigma nanoparticles, SAR$_{real(S20)}$=C$_v$.Slope$_{real(S20)}$/C$_{nano}$ for SPION20, SAR$_{real(S50)}$=C$_v$.Slope$_{real(S50)}$/C$_{nano}$ for SPION50, SAR$_{real(S100)}$=C$_v$.Slope$_{real(S100)}$/C$_{nano}$ for SPION100, where C$_v$ = 4.2 J.K$^{-1}$g$^{-1}$ is the specific heat of water and C$_{nano}$ is the nanoparticle (Magnetosome, Sigma, SPION20, SPION50, or SPION100) concentration in gram of nanoparticles per mL of water. Slope$_{real(M)}$, Slope$_{real(S)}$, Slope$_{real(S20)}$, Slope$_{real(S50)}$ Slope$_{real(S100)}$ designate the difference between the initial slope of the temperature variation with time of nanoparticles dispersed in water and the initial slope of the temperature variation with time of water without nanoparticles. For nanoparticles dispersed in water, the variation in temperature between the initial temperature measured before the application of the ultrasound and the temperature measured after 10 minutes of application of the ultrasound is designated as ΔT$_{10min(W)}$ for water alone, ΔT$_{10min(M)}$ for Magnetosomes, ΔT$_{10min(S)}$ for Sigma nanoparticles, ΔT$_{10min(S20)}$ for SPION20, ΔT$_{10min(S50)}$ for SPION50, ΔT$_{10min(S100)}$ for SPION100. The differences between ΔT$_{10min(M)}$ and ΔT$_{10min(w)}$, ΔT$_{10min(S)}$ and ΔT$_{10min(w)}$, ΔT$_{10min(S20)}$ and ΔT$_{10min(w)}$, ΔT$_{10min(S50)}$ and ΔT$_{10min(w)}$, ΔT$_{10min(S100)}$ and ΔT$_{10min(w)}$, are designated as ΔT$_{10minreal(M)}$, ΔT$_{10minreal(S)}$, ΔT$_{10minreal(S20)}$, ΔT$_{10minreal(S50)}$, ΔT$_{10minreal(S100)}$ for magnetosomes, Sigma nanoparticles, SPION20, SPION50, SPION100, respectively. The percentage in temperature rise, estimated for Sigma using the formula: Temperature rise(%)=((ΔT$_{10min(S)}$/ΔT$_{10min(W)}$)-1)*100, for Magnetosomes using the formula: Temperature rise(%)=((ΔT$_{10min(S)}$/ΔT$_{10min(W)}$)-1)*100, for SPION20 using the formula: Temperature rise(%)=((ΔT$_{10min(S20)}$/ΔT$_{10min(W)}$)-1)*100, for SPION50 using the formula: Temperature rise(%)=((ΔT$_{10min(S50)}$/ΔT$_{10min(W)}$)-1)*100, for SPION100 using the formula: Temperature rise(%) = ((ΔT$_{10min(S100)}$/ΔT$_{10min(W)}$)-1)*100.

**Table 2**

| Heating on aqueous solutions | | | |
|---|---|---|---|

| Water | 0.5 W/cm$^2$ | 1 W/cm$^2$ | 1.5 W/cm$^2$ |
|---|---|---|---|
| Slope $_{(w)}$ (°C/sec) | 0.291 | 0.467 | 0.645 |
| $\Delta T_{10\,min\,(w)}$ (°C) | 13 | 20 | 30 |

| Magnetosome | 0.5 W/cm$^2$ | 1 W/cm$^2$ | 1.5 W/cm$^2$ |
|---|---|---|---|
| Slope $_{(M)}$ (°C/sec) | 0.361 | 0.571 | 0.747 |
| Slope$_{real\,(M)}$ (°C/sec) | 0.070 | 0.104 | 0.101 |
| Slope$_{real\,N(M)}$ (mL.°C/sec/g$_{Fe}$) (mL.°C/sec/g$_{Fe}$) | 70 | 104 | 101 |
| Slope rise $_{(M)}$ (%) | 24 | 22 | 16 |
| SAR $_{(M)}$ (W/g$_{Fe}$) | 1511.3 | 2389.5 | 3124.0 |
| SAR$_{real\,(M)}$ (W/g$_{Fe}$) | 294.5 | 437.0 | 424.3 |
| $\Delta T_{10\,min\,(M)}$ (°C) | 18 | 29 | 33 |
| $\Delta T_{10\,min\,real\,(M)}$ (°C) | 5 | 9 | 3 |
| Temperature rise $_{(M)}$ (%) | 37 | 43 | 10 |

| Sigma | 0.5 W/cm$^2$ | 1 W/cm$^2$ | 1.5 W/cm$^2$ |
|---|---|---|---|
| Slope $_{(S)}$ (°C/sec) | 0.275 | 0.503 | 1.287 |
| Slope$_{real\,(S)}$ (°C/sec) | 0 | 0.036 | 0.642 |
| Slope$_{real\,N\,(S)}$ (mL.°C/sec/g$_{Fe}$) | 0 | 36 | 642 |
| Slope rise $_{(S)}$ (%) | 0 | 8 | 99 |
| SAR $_{(S)}$ (W/g$_{Fe}$) | 1150.1 | 2104.7 | 5385.6 |
| SAR$_{real\,(S)}$ (W/g$_{Fe}$) | 0 | 152.1 | 2685.9 |
| $\Delta T_{10\,min\,(S)}$ (°C) | 19 | 32 | 39 |
| $\Delta T_{10\,min\,real\,(S)}$ (°C) | 6 | 12 : | 9 |
| Temperature rise $_{(S)}$ (%) | 49 | 60 | 31 |

| SPION 50 nm | 0.5 W/cm$^2$ | 1 W/cm$^2$ | 1.5 W/cm$^2$ |
|---|---|---|---|
| Slope $_{(S50)}$ (°C/sec) | 0.274 | 0.481 | 1.313 |
| Slope$_{real\,(S50)}$ (°C/sec) | 0 | 0.015 | 0.668 |
| Slope$_{real\,N\,(S50)}$ (mL.°C/sec/g$_{Fe}$) | 0 | 15 | 668 |

| SPION 100 nm | 0.5 W/cm$^2$ | 1 W/cm$^2$ | 1.5 W/cm$^2$ |
|---|---|---|---|
| Slope $_{(S100)}$ (°C/sec) | 0.300 | 0.446 | 0.992 |
| Slope$_{real\,(S100)}$ (°C/sec) | 0.009 | 0 | 0.346 |
| Slope$_{real\,N\,(S100)}$ (mL°C/sec/g$_{Fe}$) | 9 | 0 | 346 |

| SPION 20 nm | 0.5 W/cm$^2$ | 1 W/cm$^2$ | 1.5 W/cm$^2$ |
|---|---|---|---|
| Slope $_{(S20)}$ (°C/sec) | 0.453 | 0.439 | 0833 |
| Slope$_{real\,(S20)}$ (°C/sec) | 0.162 | 0 | 0.188 |
| Slope$_{real\,N\,(S20)}$ (mL.°C/sec/g$_{Fe}$) | 162 | 0 | 188 |

(continued)

| | SPION 50 nm 0.5 W/cm² | SPION 50 nm 1 W/cm² | SPION 50 nm 1.5 W/cm² |
|---|---|---|---|
| Slope rise (S50) (%) | 0 | 3 | 104 |
| SAR (S50) (W/gFe) | 1145.5 | 2014.5 | 5495.0 |
| SAR real (S50) (W/gFe) | 0 | 62,0 | 2795.3 |
| ΔT 10 min (S50) | (°C) 15 | 26 | 34 |
| ΔT 10 min real (S50) (°C) | 2 | 5 | 4 |
| Temperature rise (S50) (%) | 18 | 27 | 14 |

| | SPION 100 nm 0.5 W/cm² | SPION 100 nm 1 W/cm² | SPION 100 nm 1.5 W/cm² |
|---|---|---|---|
| Slope rise (S100) | 3 | 0 | 54 |
| SAR (S100) (W/gFe) | 1253.2 | 1864.4 | 4149.1 |
| SAR real (S100) (W/gFe) | 36.4 | 0 | 1449,4 |
| ΔT 10 min (S100) (°C) | 17 | 27 | 36 |
| ΔT 10 min real (S100) (°C) | 4 | 7 | 6 |
| Temperature rise (S100) (%) | 34 | 35 | 20 |

| | SPION 20 nm 0.5 W/cm² | SPION 20 nm 1 W/cm² | SPION 20 nm 1.5 W/cm² |
|---|---|---|---|
| Slope rise (S20) (%) | 56 | 0 | 29 |
| SAR (S20) (W/gFe) | 1894.2 | 1837.7 | 3486.8 |
| SAR real (S20) (W/gFe) | 677.4 | 0 | 787.1 |
| ΔT 10 min (S20) (°C) | 16 | 26 | 30 |
| ΔT 10 min real (S20) | 3 | 6 | 0 |
| Temperature rise (S20) (%) | 24 | 28 | 1 |

**Table 3.** For 500 $\mu$g of magnetosomes dispersed in 100 $\mu$l of water, exposed sequentially to ultrasounds, time $t_1$ necessary to reach the desired temperature of 43$\pm$1.5 °C during the heating step (application of an ultrasound of frequency 3 MHz and power 1.5 W/cm$^2$), time $t_2$ necessary to reach 34.5$\pm$0.5 °C during the cooling step (non-application of ultrasound) during each of the 13 sequences, frequency of each sequence in mHz, $1/t_1+t_2$.

| Utrasound sequences | Time of application of the ultrasound, $t_1$ = heating step (minuntes) | Time of non-application of the ultrasound, $t_2$ = cooling step (minutes) | f (mHz) |
|---|---|---|---|
| 1 | 0.43 | 0.2 | 26 |
| 2 | 0.26 | 0.36 | 27 |
| 3 | 0.22 | 0.27 | 34 |
| 4 | 0.21 | 0.27 | 35 |
| 5 | 0.23 | 0.23 | 36 |
| 6 | 0.22 | 0.27 | 34 |
| 7 | 0.24 | 0.26 | 33 |
| 8 | 0.2 | 0.24 | 38 |
| 9 | 0.2 | 0.21 | 41 |
| 10 | 0.24 | 0.3 | 31 |
| 11 | 0.2 | 0.25 | 37 |
| 12 | 0.24 | 0.28 | 32 |
| 13 | 0.25 | 0.29 | 31 |
| Mean | 0.24 | 0.26 | 33 |

# Table 3

## Claims

1. Nanoparticles for use in an acoustic wave medical treatment of a body part of an individual, wherein the nanoparticles possess a specific absorption rate larger than 10$^{-9}$ Watt per gram of nanoparticle and the nanoparticles are administered to the body part.

2. Nanoparticles for use in an acoustic wave medical treatment of a body part of an individual, wherein the nanoparticles are administered to the body part and produce a slope of the initial variation of temperature with time, which is larger

than $10^{-9}$ °C per second per gram of nanoparticle.

3. Nanoparticles for use according to claim **1** or **2**, wherein the medical treatment is the treatment of an infectious disease, an auto-immune disease, a neuropathology, a cancer, a cutaneous condition, an endocrine disease or disorder, an intestinal disease, a communication disorder, a genetic disorder, a neurological disorder, a voice disorder, a vulvovaginal disorder, a liver disorder, a heart disorder, a heating disorder, a mood disorder, or a personality disorder.

4. Nanoparticles for use according to any one of claim **1** or **3**, wherein the nanoparticles are crystallized, metallic, or magnetic.

5. Nanoparticles for use according to any one of claim **1** to **4**, wherein the thermal conductivity or density of the body part, the velocity of the acoustic wave, attenuation of the acoustic wave, absorption of the acoustic wave, elasticity of the acoustic wave, or acoustic impedance of the acoustic wave, is at least 1.1 larger or lower in the body part comprising the nanoparticles than in the body part without the nanoparticles.

6. Nanoparticles for use according to any one of claim **1** to **5**, wherein the nanoparticles are administered to the body part and the body part is sequentially exposed to acoustic waves of intensity lower than 1000 W/cm$^2$.

7. Nanoparticles for use according to any one of claim **1** to **6**, wherein the acoustic wave is an ultrasound.

8. Nanoparticles for use according to any one of claim **1** to **7**, wherein the frequency of the acoustic wave is lower than 100 MHz.

9. Nanoparticles for use according to any one of claim **1** to **8**, wherein the acoustic wave has a penetration depth in the body part, which is larger than 1 cm.

10. Nanoparticles for use according to any one of claim **1** to **9**, wherein the concentration of the administered nanoparticles is larger than $10^{-3}$ mg per mm$^3$ of body part.

11. Nanoparticles for use according to any one of claim **1** to **10**, wherein the nanoparticles induce the destruction of a pathological site of the body part without the destruction of a healthy site surrounding the pathological site.

12. Nanoparticles for use according to any one of claim **1** to **11**, wherein the application of the acoustic wave on nanoparticles induces a temperature increase in the pathological site of more than 1 °C and/or a temperature increase in the healthy site surrounding the pathological site of less than 100 °C.

13. Nanoparticles for use according to any one of claim **1** to **12**, wherein the application of acoustic waves on the body part leads to a temperature increase of the body part, which is at least 1% larger in the presence than in the absence of the nanoparticles.

14. Nanoparticles for use according to any one of claims **1** to **13**, wherein the application of the acoustic wave on nanoparticles induces a series of temperature increases of the body part followed by temperature decreases of the body part.

15. Nanoparticles for use according to any one of claim **1** to **14**, wherein the temperature increase of the body part occurs within less than 50% of the body part.

16. Nanoparticles for use according to any one of claim **1** to **15**, wherein the temperature increase of the body part occurs within more than 1% of the volume occupied by the nanoparticles in the body part.

**Figure 1**

**Figure 2**

**Figure 3**

Figure 4

Europäisches
Patentamt

European
Patent Office

Office européen
des brevets

# EUROPEAN SEARCH REPORT

Application Number

EP 17 02 0555

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/335156 A1 (KOSHELEVA OLGA K [TW] ET AL) 13 November 2014 (2014-11-13) * paragraphs [0010], [0012], [0021] - [0022], [0046], [0068], [0071], [0077], [0096] * ----- | 1-16 | INV. A61K41/00 |
| A | WO 2011/061259 A1 (NANOBACTERIE [FR]; ALPHANDERY EDOUARD [FR]; FAURE STEPHANIE [FR]; CHEB) 26 May 2011 (2011-05-26) * page 1, paragraph 1 * ----- | 1-16 | |

TECHNICAL FIELDS
SEARCHED (IPC)

A61K

The present search report has been drawn up for all claims

| Place of search | Date of completion of the search | Examiner |
|---|---|---|
| The Hague | 30 May 2018 | Siebum, Bastiaan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&: member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 17 02 0555

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2018

| Patent document cited in search report | | Publication date | Patent family member(s) | | Publication date |
|---|---|---|---|---|---|
| US 2014335156 | A1 | 13-11-2014 | TW | 201440789 A | 01-11-2014 |
| | | | US | 2014335156 A1 | 13-11-2014 |
| | | | US | 2015328485 A1 | 19-11-2015 |
| WO 2011061259 | A1 | 26-05-2011 | AU | 2010320918 A1 | 07-06-2012 |
| | | | BR | 112012011767 A2 | 27-03-2018 |
| | | | CA | 2780851 A1 | 26-05-2011 |
| | | | CN | 102811726 A | 05-12-2012 |
| | | | EP | 2370085 A1 | 05-10-2011 |
| | | | ES | 2539588 T3 | 02-07-2015 |
| | | | IL | 219745 A | 21-04-2016 |
| | | | JP | 6202358 B2 | 27-09-2017 |
| | | | JP | 2013511491 A | 04-04-2013 |
| | | | JP | 2016155821 A | 01-09-2016 |
| | | | RU | 2012122639 A | 27-12-2013 |
| | | | US | 2012302819 A1 | 29-11-2012 |
| | | | US | 2017157253 A1 | 08-06-2017 |
| | | | WO | 2011061259 A1 | 26-05-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- FR 2016000095 W